(19) **Europäisches Patentamt / European Patent Office / Office européen des brevets**

(11) **EP 1 489 559 B1**

(12) **EUROPEAN PATENT SPECIFICATION**

(45) Date of publication and mention
of the grant of the patent:
**10.10.2007  Bulletin 2007/41**

(51) Int Cl.:
***G06T 11/00*** *(2006.01)*

(21) Application number: **04253585.6**

(22) Date of filing: **16.06.2004**

(54) **Cone-beam reconstruction apparatus and computed tomography apparatus**

Vorrichtung zur Rekonstruktion von Kegelstrahlprojektionsdaten und Vorrichtung zur Computertomografie

Dispositif pour la reconstruction de données à faisceau conique et dispositif de tomographie par ordinateur

(84) Designated Contracting States:
**DE FR GB NL**

(30) Priority: **16.06.2003  US 461401**

(43) Date of publication of application:
**22.12.2004  Bulletin 2004/52**

(73) Proprietors:
- **KABUSHIKI KAISHA TOSHIBA**
  **Tokyo 105-8001 (JP)**
- **Toshiba Medical Systems Corporation**
  **Otawara-shi,**
  **Tochigi-ken 324-8550 (JP)**

(72) Inventors:
- **Taguchi, Katsuyuki**
  **Buffalo Grove 60089,**
  **Illinois (US)**
- **Chiang, Be-Shan Su**
  **Buffalo Grove 60089,**
  **Illinois (US)**

(74) Representative: **Midgley, Jonathan Lee**
**Marks & Clerk**
**90 Long Acre**
**London WC2E 9RA (GB)**

(56) References cited:
**DE-A- 10 244 181       US-B1- 6 408 042**

- TAGUCHI K ET AL: "A new weighting scheme for cone-beam helical CT to reduce the image noise" PHYSICS IN MEDICINE AND BIOLOGY IOP PUBLISHING UK, vol. 49, no. 11, 19 May 2004 (2004-05-19), pages 2351-2364, XP002299943 ISSN: 0031-9155
- STIERSTORFER K ET AL: "Weighted FBP-a simple approximate 3D FBP algorithm for multislice spiral CT with good dose usage for arbitrary pitch" PHYSICS IN MEDICINE AND BIOLOGY IOP PUBLISHING UK, vol. 49, no. 11, 19 May 2004 (2004-05-19), pages 2209-2218, XP002299944 ISSN: 0031-9155
- FELDKAMP L A ET AL: "Practical cone-beam algorithm" JOURNAL OF THE OPTICAL SOCIETY OF AMERICA - A, OPTICAL SOCIETY OF AMERICA, WASHINGTON, US, vol. 1, no. 6, 1 June 1984 (1984-06-01), pages 612-619, XP002085783 ISSN: 1084-7529

**Description**

Multi-slice X-ray computed tomography (CT) systems were developed and introduced into the medical market in 1998. Generally, the number of slices used in a multi-slice X-ray CT ranges from about 2 to about 16. However, the number of slices is expected to increase. Some expect the number of slices used to increase to 32, 64, or even perhaps 256. (See references Y. Saito, H. Aradate, H. Miyazaki, K. Igarashi, and H. Ide, "Development of a large area 2-dimensional detector for real-time 3-dimensional CT (4D-CT)," Radiology vol. 217(P), 405 (2000); Y. Saito, H. Aradate, H. Miyazaki, K. Igarashi, and H. Ide, "Large area two-dimensional detector system for real-time three-dimensional CT (4D-CT)," Proc. of SPIE Med. Imag. Conf., 4320, 775-782 (2001); and http://www3.toshiba.co jp/medical/4d-ct/).

Several imagery construction algorithms are used for helical scanning in a multi-slice X-ray CT system. One such image reconstruction algorithm uses a generalized weighted version of a Feldkamp reconstruction algorithm. (See L. A. Feldkamp, L. C. Davis, and J. W. Kress, "Practical cone-beam algorithm," J. Opt. Soc. Am. A, 6, 612-19 (1984); H. Aradate and K. Nambu, "Computed tomography apparatus," Japanese Patent No. 2,825,352; L. G. Zeng and G. T. Gullberg, "Short-scan cone beam algorithm for circular and noncircular detector orbit," Proc. of SPIE Med. Imag. Conf, 1233, 453-463 (1990); H. Kudo and T. Saito, "Three-dimensional helical-scan computed tomography using cone-beam projections," J. Electron. Information Commun. Soc. Japan, J74-D-II, 1108-1114 (1991); G. Wang, T. H. Lin, P. C. Cheng, D. M. Shinozaki, "A general cone-beam reconstruction algorithm," IEEE Trans. Med. Imaging, 12, 486-496 (1993); K. Taguchi, "X-ray computed tomography apparatus," U.S. Patent No. 5,825,842 (Filed in 1995); K. Wiesent, K. Barth, N. Novab, et al., "Enhanced 3-D-reconstruction algorithm for C-arm systems suitable for interventional procedures," IEEE Trans. Med. Imaging, 19, 391-403 (2000); M. D. Silver, K. Taguchi, and K. S: Han, "Field-of-view dependent helical pitch in multi-slice CT," Proc. of SPIE Med. Imag. Conf., 4320, 839-850 (2001); M. D. Silver, K. Taguchi, and I. A. Hein, "A simple algorithm for increased helical pitch in cone-beam CT," The Sixth International Meeting on Fully Three-Dimensional Image Reconstruction in Radiology and Nuclear Medicine, 70-73 (2001)). The generalized weighted version of the Feldkamp reconstruction algorithm introduces a flexible focus orbit and applies a weighting function to the Feldkamp algorithm. Specifically, the algorithm applies weight to projection data prior to 1-dimensional (1D) filtering (or convolution) and 3-dimensional (3D) backprojection.

The weighting function may be one developed for 2-dimensional (2D) fan-beam reconstruction. Using the weighting function developed for 2D fan-beam reconstruction, however, the same weight is applied to all detector rows. In other weight is independent of cone-angle, even though the projection data in the cone-beam CT is diversion in detector row (z) direction with cone-angle.

US 6 408 042 describes a method for cone-beam artifact suppression in scanning imaging systems where a cone-angle dependent weighting function is used.

As the number of examinations using multi-slice CT increases, the X-ray exposure in CT scanning has become more of a concern. As a result, physicians physicists have increased their efforts to reduce the patient dose while keeping image noise constant. Additionally, physicists and physicians seek to decrease the image noise using the same data.

Accordingly, an object of this invention is to provide a method, system, and computer program product for improved multi-slice X-ray computer tomography systems using a detector row dependent weight to the generalized weighted cone-beam backprojection. One example of a backprojection technique to which the invention is particularly applicable is the helical Feldkamp algorithm. Some useful techniques of Feldkamp reconstruction may be found in (L.A. Feldkamp, L.C. Davis, and J.W. Kress, "Practical Cone-Beam "Algorithm," Journal Optical Society of America, Vol. 1, pp 612-619 (1984)).

This and other objects are achieved by a way of a method, system, and computer program product constructed according to the present invention.

In a first aspect, the present invention provides an X-ray CT apparatus, characterized by comprising: a helical scanning device configured to collect projection data while at least one of a gantry and a couch moves along an axial direction of the couch, the helical scanning device including, an X-ray source configured to generate X-rays, and a detector having detector elements arranged in a plurality of rows along the axial direction and configured to produce the projection data; and a processor comprising, a weighted device configured to apply a weighting function including a cone-angle dependent weight to the projection data, thereby obtaining weighted data, a filtering device configured to filter the weighted data, and a backprojecting device configured to backproject the weighted data taking cone-angle into account, the apparatus being configured such that projection data whose cone angle is outside a predetermined cone angle ($\alpha 0$) is not measured, the non-measured projection data is generated by extrapolation or copying and the generated projection data is weighted according to the cone angle.

In a second aspect, the present invention provides a method for processing data obtained from a CT scan, said CT scan being performed an X-ray CT apparatus, comprising:

a helical scanning device configured to collect projection data while at least one of a gantry and a couch moves along an axial direction of the couch, the helical scanning device including,

an X-ray source configured to generate X-rays, and
a detector having detector elements arranged in a plurality of rows along the axial direction and configured to produce the projection data,
said method comprising:

applying a weighting function including a cone-angle dependent weight to the projection data, thereby obtaining weighted data,
filtering the weighted data, and
backprojecting the weighted data taking cone-angle into account,

wherein the projection data whose cone angle is outside a predetermined cone angle is not measured, the non-measured projection data is generated by extrapolation or copying and the generated projection data is weighted according to the cone angle.

Thereby, the projection data range is increased without sacrificing image quality (without introducing additional artefacts). Through the present invention, it is possible to decrease the image noise from an identical data set. Simply put, it is possible to use noisier data with a smaller patient dose to achieve the same output image.

This summary of the invention does not necessarily describe all necessary features so that the invention may also be a sub-combination of these described features.

The invention can be more fully understood from the following detailed description when taken in conjunction with the accompanying drawings, in which:

Fig. 1a illustrates an example with the current weight at $\gamma=0$.
Fig. 1b also illustrates the current weighting method.
Fig. 1c is illustrative of the weighting method of the present invention.
Fig. 2 illustrates Cone angle (or "validity of data") dependent weight, $^{Cone}w$.
Figs. 3a and 3b illustrate primary and complementary rays.
Fig. 4 illustrates cases when the $\alpha_c$ calculated becomes strange value.
Fig. 5 illustrates geometry of backprojection, specifically a projection angle, $\beta$, a ray-angle, $\gamma$, and a virtual fan-angle, $\Gamma_m$.
Fig. 6 depicts a weighting function for Extended Half-Scanning (MHS) applied to each segment of multiple views to be backprojected.
Fig. 7 represents a weighting function for overscanning (hereafter, OS).
Fig. 8a illustrates the parameters related to the cone angle, $\alpha$, $\alpha_0$, and $\alpha_m$.
Fig. 8b depicts weighting as a function of cone angle.
Fig. 9 represents a weighting function of OS.
Figs. 10a, 10b, 10c, 10d, and 10e show direct and complementary rays.
Figs. 11a-11e illustrate results using the OS method.
Figs. 12a-12e and 13a-13e illustrate results using the current OS method.
Fig. 14 depicts the method of using six complementary rays all the time.
Fig. 15 depicts the method using only valid complementary rays.
Fig. 16 illustrates a weighting function of MHS.

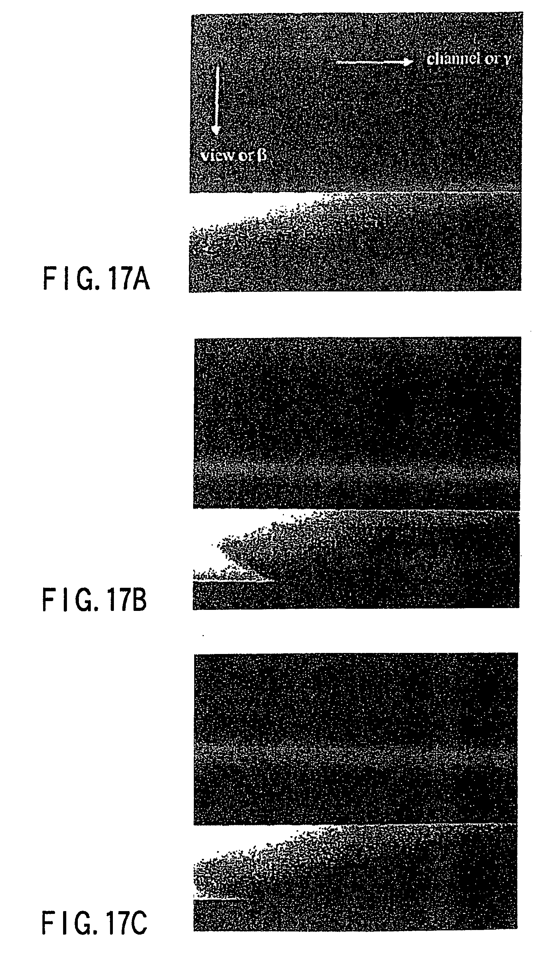

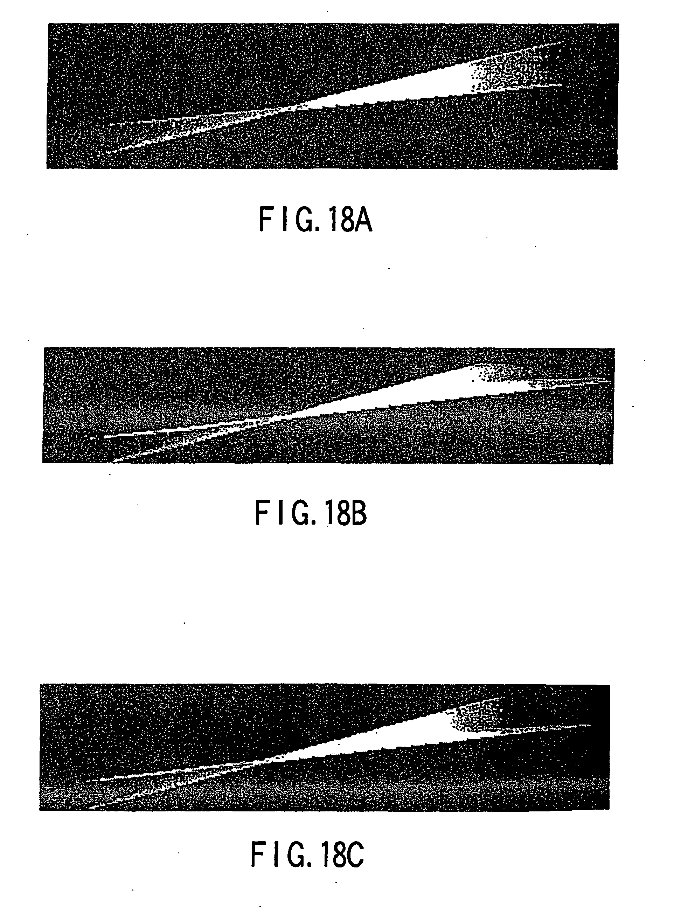

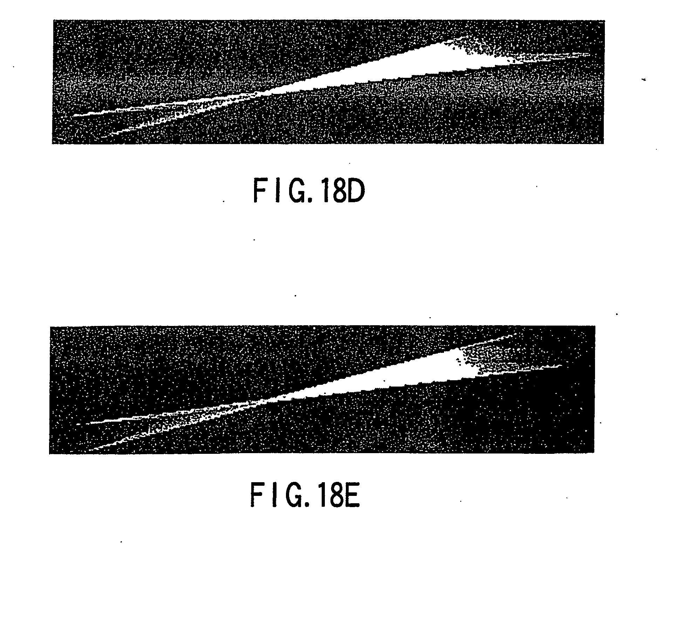

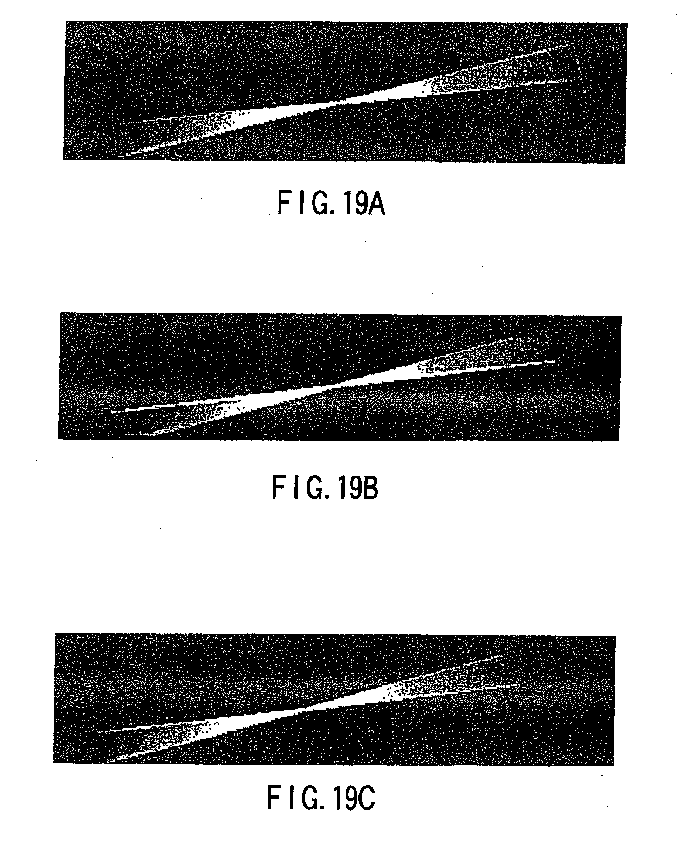

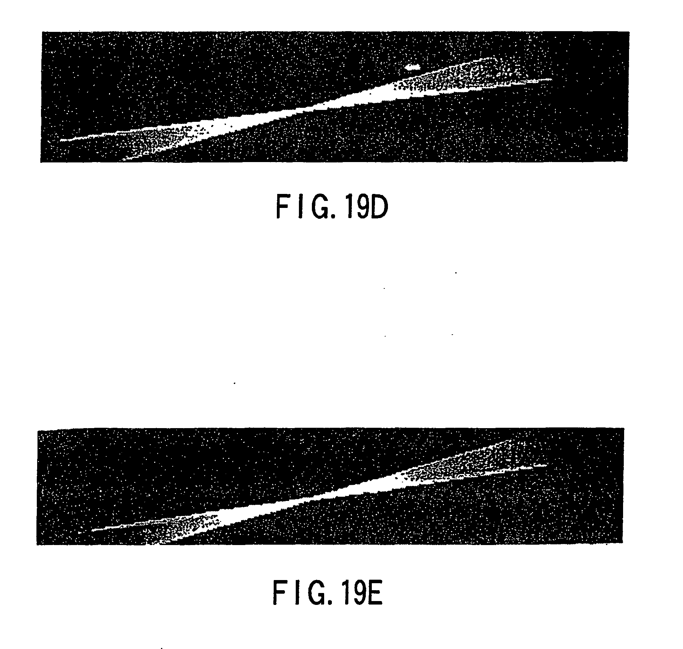

Figs. 17a-17c, 18a-18e, and 19a-19e illustrate results using the current MHS method.
Fig. 20 illustrates a view showing the configuration of the X-rays computed tomography system according to this embodiment of the invention.

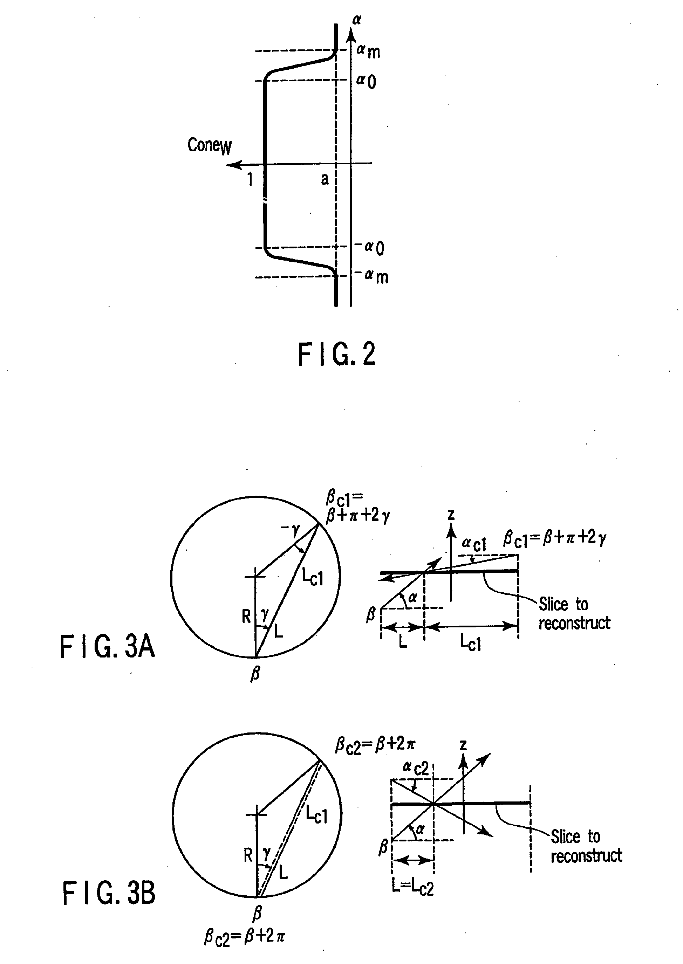

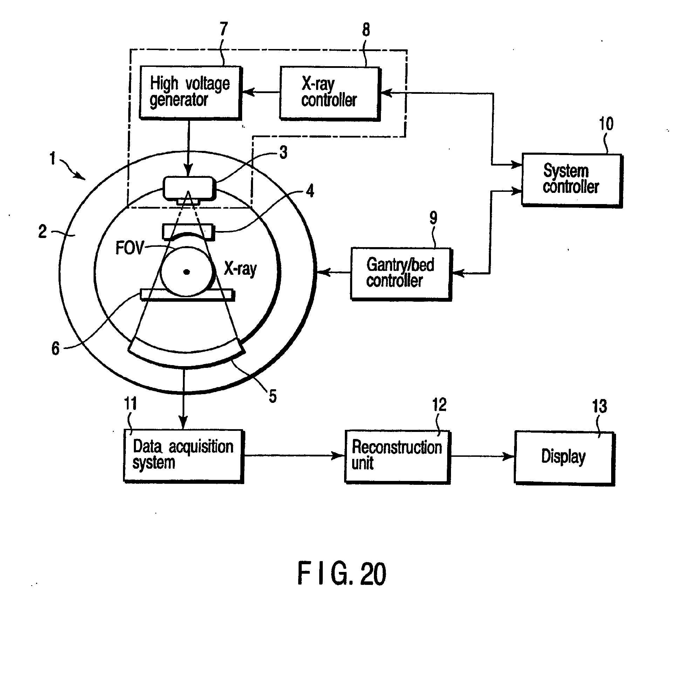

Referring now to the drawings, wherein like reference numerals designate identical or corresponding parts throughout the several views. Fig. 20 shows the configuration of the X-rays computed tomography system according to this embodiment of the invention. The projection data measurement system constituted by gantry 1 accommodates an x-ray source 3 that generates a cone-beam of x-ray flux approximately cone-shaped, and a two-dimensional array type x-ray detector 5 consisting of a plurality of detector elements 5A arranged in two-dimensional fashion, i.e., a plurality of elements arranged in one dimension stacked in a plurality of rows. In FIG. 3, ten rows each having 1000 elements are shown (other arrangements are possible), with the x-ray flux shown schematically emitted from focal point F. X-ray source 3 and two-dimensional array type x-ray detector 5 are installed on a rotating ring 2 in facing opposite sides of a subject, who is laid on a sliding sheet of a bed 6. Two-dimensional array type x-ray detector 5 is mounted on rotating ring 2. Each detector element will correspond with one channel. X-rays from x-ray source 3 are directed on to subject through an x-ray filter 4. X-rays that have passed through the subject are detected as an electrical signal by two-dimensional array type x-ray detector 5. X-ray controller 8 supplies a trigger signal to high voltage generator 7. High voltage generator 7 applies high voltage to x-ray source 3 with the timing with which the trigger signal is received. This causes x-rays to be emitted from x-ray source 3. Gantry/bed controller 9 synchronously controls the revolution of rotating ring 2 of gantry 1 and the sliding of the sliding sheet of bed 6. System controller 10 constitutes the control center of the entire system and controls x-ray controller 8 and gantry/bed controller 9 such that, as seen from the subject, x-ray source 3 executes so-called helical scanning, in which it moves along a helical path. Specifically, rotating ring 2 is continuously rotated with fixed angular speed while the sliding plate is displaced with fixed speed, and x-rays are emitted continuously or intermittently at fixed angular intervals from x-ray source 3. The output signal of two-dimensional array type x-ray detector 5 is amplified by a data collection unit 11 for each channel and converted to a digital signal, to produce projection data. The projection data that is output from data collection unit 11 is fed to reconstruction processing unit 12. Reconstruction processing unit 12 uses the projection data to find backprojection data reflecting the x-ray absorption in each voxel. In the helical scanning system using a cone-beam of x-rays as in the first embodiment, the imaging region (effective field of view) is of cylindrical shape of radius .omega. centered on the axis of revolution. Reconstruction processing unit 12 defines a plurality of voxels (three-dimensional pixels) in this imaging region, and finds the backprojection data for each voxel. The three-dimensional image data or tomographic image data compiled by using this backprojection data is sent to display device 14, where it is displayed visually as a three-dimensional image or tomographic image.

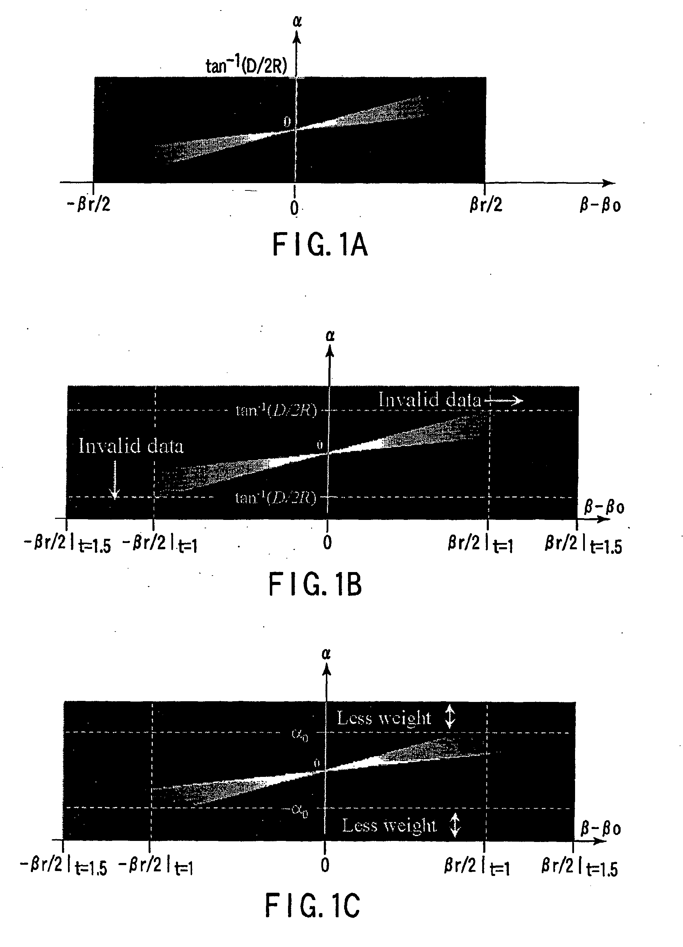

Fig. 1a shows an example of the weighting at r = 0 in the related art. One of the problems of using the weighting is that when the projection angular range (as shown in Figs. 1a and 1b) is extended, replicated invalid data have the same weight. Using such invalid data degrades image quality by introducing artifacts and increasing noise if an extrapolation method is used.

In light of the deficiencies of the related art method, the Applicants have discovered another weighting application as a function of the validity of the projection data. This weighted data is then combined with the overscan or extended half-scan methods, depending on the helical pitch. The newly discovered weighting method permits extension of the projection angular range for reconstruction, while minimizing the side effects of extrapolation or replication, as shown in Fig. 1c.

The following parameters are useful when performing the newly discovered weighting method of the present invention:

$\beta, \gamma, \alpha$: projection angle, ray angle, and cone angle for direct ray;
$\beta_c, \gamma_c, \alpha_c$: projection angle, ray angle, and cone angle for complementary ray;
$\gamma_m, \Gamma_m$: physical and virtual fan angle;
$\alpha_0$ Tuning parameter-1: cone angle to start decreasing weight;
$\alpha_m$ Tuning parameter-2: $\alpha_0 < \alpha_m$ (width for weighting curve);
a Tuning parameter-3: $0 < a < 1$ (height of weighting curve);
1 Table feed per one rotation [mm/rev];
R Focus to rotation axis distance [mm];
r The radius of maximum field of view [mm];
L, $L_c$ Focus to voxel distance projected onto the xy plane; and
z, $z_c$ The z coordinate of the focus position.

Generally, cone-beam projections measured along a helical orbit are given by the equations:

$$g(\beta, \gamma, \alpha) = \int_0^\infty f\left(\bar{s}(\beta) + l\bar{\varphi}_{\beta,\gamma,\alpha}\right) dl \qquad (1)$$

$$\bar{s}(\beta) = \left(R\cos\beta, R\sin\beta, H \cdot \beta/2\pi\right)^T; \quad 0 \le \beta \le 2\pi n, \qquad (2)$$

where $f(\bar{r})$ is the object to reconstruct, R is the radius of the helical orbit, H is the helical pitch, $(\beta\gamma\alpha)$ denote projection angle, ray angle, and cone angle, respectively, and $\bar{\varphi}_{\beta,\gamma,\alpha}$ denotes the unit vector, which is directed from the x-ray focus $\bar{s}(\beta)$ toward the point $(\gamma, \alpha)$ on the circular arc-shaped detector surface at $\beta$. Current experiments indicate that combination of $\alpha_0$ corresponding to slightly inside of the edge of the detector and $\alpha_m$ slightly outside of the edge achieve the best results.

Current Weighted Feldkamp Helical Algorithm

The present embodiment will now be described applying the generalized weighted Feldkamp algorithm. However, other backprojection techniques are equally applicable, and the following description based on the Feldkamp algorithm should not be deemed to preclude the application of the present invention to other techniques of backprojection.

The generalized weighted Feldkamp algorithm includes the following three steps: 1) applying a weighting function to the projection data (applying the same weight to all of the detector rows), 2) filtering the data in the horizontal direction(or a filtering along the direction such as the tangent direction of the helical path), and 3) cone-beam backprojection.

Step 1: Weighting

$$\tilde{g}(\beta,\gamma,\alpha) = \cos \zeta(\gamma,\alpha) \cdot w(\beta - \beta_0,\gamma,\alpha) \cdot g(\beta,\gamma,\alpha), \tag{3}$$

$$\cos \zeta(\gamma,\alpha) = \bar{\varphi}_{\beta,\gamma,\alpha} \cdot \bar{\varphi}_{\beta,0,0}, \tag{4}$$

where $w(\beta,\gamma,\alpha)$ denotes the weighting function (discussed later) and $\beta_0$ refers to the center of data range used in reconstruction ($\beta_r$).

Steps 2 and 3: Filtering and Cone-Beam Backprojection

$$f(\bar{r}) = \frac{1}{2\pi} \int_{\beta_0 - \beta_r/2}^{\beta_0 + \beta_r/2} \frac{R \|\bar{s}'(\beta)\|}{[(\bar{r} - \bar{s}(\beta)) \cdot \bar{\varphi}_{\beta,0,0}]^2} \int_{-\infty}^{\infty} [h(\gamma - \gamma') \cdot \tilde{g}(\beta,\gamma',\alpha)] d\gamma' \, d\beta, \tag{5}$$

$$\beta_r = \frac{2\pi}{H/D} \times \left(1 - \frac{r_0}{R}\right), \tag{6}$$

where the function $h(\cdot)$ denotes the filter function (e.g., ramp, Shepp-Logan, or the like), $r_o$ represents the radius of the cylindrical support of the object, and D represents the detector height at the iso-center (width in the z direction of the detector on the rotation center axis (z axis) ).

Once the backprojection view range is defined, the projection view data for the range is weighted (using techniques such as those described in Dennis L. Parker, "Optimal Short Scan Convolution Reconstruction for Fanbeam CT," Med. Phys. 9(2), March/April 1982), and convolved (using techniques analogous to those described in H.H. Barrett and W. Swindell, Radiological Imaging: Theory of Image Formation, Detection, and Processing, Vol. 2, New York: Academic Press, pp 391-392 (1981)) as desired prior to backprojection.

Weighting functions used for helical scanning vary depending on helical pitch. Generally, over-scan functions are used for small helical pitch (projection range $\beta_r > 2\pi$) and extended half-scan for high helical pitch ($\beta_r \le 2\pi$) (See, e.g., M. D. Silver, K. Taguchi, and K. S. Han, "Field-of-view dependent helical pitch in multi-slice CT," Proc. of SPIE Med. Imag. Conf., 4320, 839-850 (2001); M. D. Silver, K. Taguchi, and I. A. Hein, "A simple algorithm for increased helical pitch in cone-beam CT," The Sixth International Meeting on Fully Three-Dimensional Image Reconstruction in Radiology and Nuclear Medicine, 70-73 (2001); C. R. Crawford and K. F. King, "Computed tomography scanning with simultaneous patient translation," Med. Phys. 17, 967-982 (1990); M. D. Silver, "A method for including redundant data in computed tomography," Med. Phys. 27, 773-774 (2000); and D. L. Parker, "Optimal short scan convolution reconstruction for fanbeam CT," Med. Phys. 9, 254-257 (1982).)

In the over-scan functions, weight is a function of projection angle, $\beta$ as shown in Equations 7-10 below:

$$^{os}w_\beta = \left(3x_\beta^2 - 2x_\beta^3\right)/2, \tag{7}$$

$$x_\beta = \begin{cases} \dfrac{\beta'}{\lambda_{os}} & (0 \le \beta' < \lambda_{os}) \\ 1 & (\lambda_{os} \le \beta' < 2\pi) \\ \dfrac{2\pi + \lambda_{os} - \beta'}{\lambda_{os}} & (2\pi \le \beta' \le 2\pi + \lambda_{os}) \\ 0 & (otherwise) \end{cases}, \tag{8}$$

$$\beta' = \beta - (\beta_0 - \beta_r/2) = \beta - (\beta_0 - (2\pi + \lambda_{os})/2), \tag{9}$$

$$\lambda_{os} = \beta_r - 2\pi. \tag{10}$$

In the extended half-scan functions, weights are a function of ray angle, $\gamma$, as well as projection angle, $\beta$, as shown in Equations 11-14 below:

$$^{HS}w_{\beta,\gamma} = 3x^2_{\beta,\gamma} - 2x^3_{\beta,\gamma}, \tag{11}$$

$$x_{\beta,\gamma} = \begin{cases} \dfrac{\beta'}{2(\Gamma - \gamma)} & (0 \le \beta' < 2(\Gamma - \gamma)) \\ 1 & (2(\Gamma - \gamma) \le \beta' < \pi - 2\gamma) \\ \dfrac{\pi + 2\Gamma - \beta'}{2(\Gamma + \gamma)} & (\pi - 2\gamma \le \beta' \le \pi + 2\Gamma) \\ 0 & (otherwise) \end{cases}, \tag{12}$$

$$\beta' = \beta - (\beta_0 - \beta_r/2) = \beta - (\beta_0 - (\pi + 2\Gamma)/2), \tag{13}$$

$$2\Gamma = \beta_r - \pi. \tag{14}$$

One known weighting algorithm uses pixel dependent weighting during cone-beam backprojection, as well as pixel dependent projection angular range, which is often too complicated to implement. (See, e.g., Hu et al., U. S. Pat. No. 5,430,783.)

Another known weighting algorithm modifies extended half-scan by applying weight as a function of "pure cone-angle" to the projection data, modifying the projection data, and normalizing the weights of primary and complementary rays. (See, e.g., S. K. Patch, A. Nishide, A. Hagiwara, "Volumetric computed tomography data weights - Resolution vs artifact," Radiology 225(P), 496 (2002)).

$$x(\alpha, \beta, \gamma) = \frac{P(\beta, \gamma)^P \cdot (\cot \alpha)^{2a}}{P(\beta, \gamma)^P \cdot (\cot\alpha)^{2a} + P(\beta_c, \gamma_c)^P \cdot (\cot\alpha_c)^{2a}}, \tag{15}$$

where a is a parameter for tuning the algorithm and P is the normalized helical pitch.

Rewriting Equation 15 to obtain uniform notation, Equation 15 becomes:

$$^{ConeMHS}w_{\beta,\gamma,a} = \frac{\left(^{MHS}w_{\beta,\gamma,a}\right)^{H/D} \cdot (\cot\alpha)^{2a}}{\left(^{MHS}w_{\beta,\gamma,a}\right)^{H/D} \cdot (\cot\alpha)^{2a} + \left(^{MHS}w_{\beta,\gamma,a}\right)^{H/D} \cdot (\cot\alpha_c)^{2a}} \tag{16}.$$

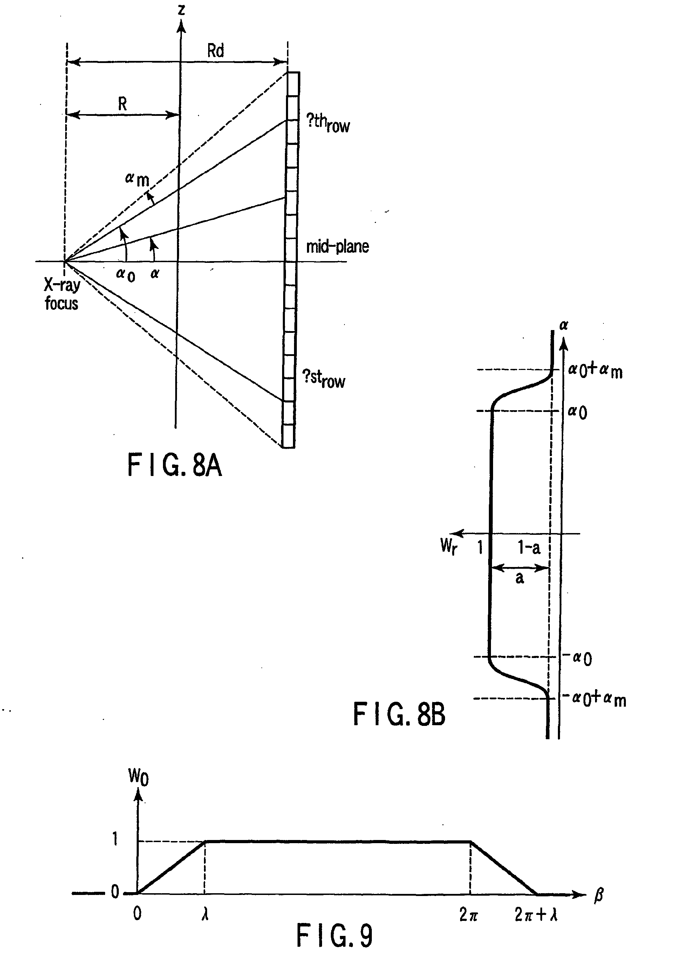

However, in the formula of Equations 15 and 16, altering the cone angle (cotα or altering the original weight as a function of normalized helical pitch does not sufficiently reduce the effect of extrapolation or replication when the cone angle α is small. Additionally, the method of Equations 15 and 16 does not consider the validity (or potential invalidity) of the data being used. It is also important to note that the parameter "a" used in Equation 16 is distinct from the a of Fig. 8b.

Fig. 1a shows an example of the current weight at γ = 0. One problem of the current method is that extending the projection angular range (as shown in Figs. 1a and 1b) requires the use of extrapolated (or replicated) invalid data having the same weight as the valid (measured) data (Fig. 1b). Using such invalid data degrades image quality by introducing artifacts (and increasing noise if extrapolation is used).

Therefore, in light of the above-described difficulties, the present invention relates to applying another weight as a function of the validity of projection data and combining the other weight with overscan or extended half-scan, depending on helical pitch. Therefore, it is possible to extend the projection angular range for reconstruction while minimizing side effects of extrapolation and replication (Fig. 1c). If $\beta_r > 4\pi$, another weighting function should be applied that normalizes the weights to the primary and complementary rays. One such weighting function is shown below in Equation (D1):

$$rot = floor \frac{N}{N_{360}}$$

$$overlap = N - N_{360} \times rot$$

$$If(i == 0)$$

$$Error. \; Should \; be \; MHS \; instead \; of \; OS$$

$$elseif(i == 1)$$

$$w_0 = 1/rot$$

$$weight(n) = \begin{cases} \dfrac{n}{overlap} & 0 \le n < overlap \\ 1 & overlap \le n < overlap + N_{360} \times rot \;, \\ \dfrac{N-n-1}{overlap} & overlap + N_{360} \times rot \le n \le N-1 \end{cases} \quad (D1)$$

where $N_{360}$ is the number of views per rotation and $N$ is the number of preferred views for one slice.

First, the projection range of Equation (6) is enlarged to be:

$$\beta_r = \frac{2\pi}{H/(tD)} \times \left(1 - \frac{r_0}{R}\right); t > 1. \quad (17)$$

Cone-angle Dependent Weight: $^{row}w$

The data validity weighting function is set forth in equations 18-20, below:

$$^{Cone}w = a + (1-a) \cdot \left(3x_\alpha^2 - 2x_\alpha^2\right), \quad (18)$$

$$x_\alpha = \begin{cases} 1 & (|\alpha| \le \alpha_0) \\[2mm] \dfrac{\alpha_m - \alpha}{\alpha_m - \alpha_0} & (\alpha_0 < |\alpha| \le \alpha_m), \\[2mm] 0 & (\alpha_m < |\alpha|) \end{cases} \tag{19}$$

and

$$\alpha_m > \tan^{-1} \frac{D}{2R}. \tag{20}$$

Here, two cone-angles ($\alpha_0$ and $\alpha_m$) define the modification point of the validity curve (as shown in Fig. 2). In the equation, a is a cone angle at which the line connecting a specific ray of the cone beam and the focal point of the cone beam intersects with the plane perpendicular to the rotation axis (i.e., x axis), and $\alpha0$ is a cone angle for the ray applied to a detecting element near one end of any element-row of the detector array. The cone angle $\alpha0$ is not limited in particular. Therefore, it can be of the value for the ray applied to one end of the element-row. Nonetheless, as known in the art, the cone angle $\alpha0$ should better have an absolute value slightly smaller than the cone angle for the ray applied to one end of the element-column. The cone angle $\alpha m$ has an absolute value a little greater than the angle $\alpha0$. The channel corresponding to the angle $\alpha m$ or $\alpha0$ may be an outermost one. Nevertheless, as is known in the art, too, it is desired that either outermost channel should lie between the lines corresponding to the cone angles $\alpha0$ and $\alpha m$. A part of the data, which is not actually measured of the channels lying between the lines corresponding to the angles $\alpha0$ and $\alpha m$, has been generated by extrapolation or is a copy of the data obtained at either outermost channel. The data obtained at some of the channels lying between the lines corresponding to the cone angles $\alpha0$ and $\alpha m$ and the data obtained outside the line corresponding to the angle $\alpha m$, which have not been actually measured, are not so reliable. They are therefore weighted less than the actually measured data.

Complementary Ray: $d^C(\beta,\gamma,\alpha) = d^P(\beta_c,\gamma_c,\alpha_c)$

For each "primary ray ($d^P(\beta,\gamma,\alpha)$)," it is possible to find corresponding "complementary rays ($d^{C(n)}(\beta,\gamma,\alpha)$)" whose projected path onto the xy plane coincides with that of the primary ray. Specifically, the primary and complementary rays intersect the same point in the slice of interest (as illustrated in Figs. 3a and 3b). The number of complementary rays may vary from 1 to 4, depending on $\beta_r$.

Functions for Complementary Rays

Projection angle and ray angle

$$\beta_c = \begin{cases} \beta + \pi + 2\gamma & (\beta + \pi + 2\gamma \le \pi) \\[2mm] \beta - \pi + 2\gamma & (otherwise) \end{cases} \tag{B1}$$

$$\gamma_c = -\gamma \tag{B2}$$

Cone angle

The z coordinates of each focus and the cone angle to the voxel of interest are defined by:

$$z = -\frac{\beta}{2\pi} \cdot l; \quad z_c = -\frac{\beta_c}{2\pi} \cdot l, \text{ and} \tag{B3}$$

$$\alpha = \tan^{-1}\frac{z}{L}; \quad \alpha_c = \tan^{-1}\frac{z_c}{L_c}. \tag{B4}$$

Thus,

$$L = \frac{z}{\tan\alpha}; \quad L_c = \frac{z_c}{\tan\alpha_c}. \tag{B5}$$

From Figs. 3a and 3b:

$$L + L_c = 2R\cos\gamma. \tag{B6}$$

Using the above equations:

$$\alpha_c = \tan^{-1}\frac{-\beta_c/_{2\pi}\cdot l}{2R\cos\gamma - L}$$

$$= \begin{cases} \tan^{-1}\left[\dfrac{-l\cdot\tan\alpha\cdot(\beta+\pi+2\gamma)}{4\pi R\cdot\tan\alpha\cdot\cos\gamma+\beta\cdot l}\right] & (\beta+\pi+2\gamma\le\pi) \\[2em] \tan^{-1}\left[\dfrac{-l\cdot\tan\alpha\cdot(\beta-\pi+2\gamma)}{4\pi R\cdot\tan\alpha\cdot\cos\gamma+\beta\cdot l}\right] & (otherwise) \end{cases} \tag{B7}$$

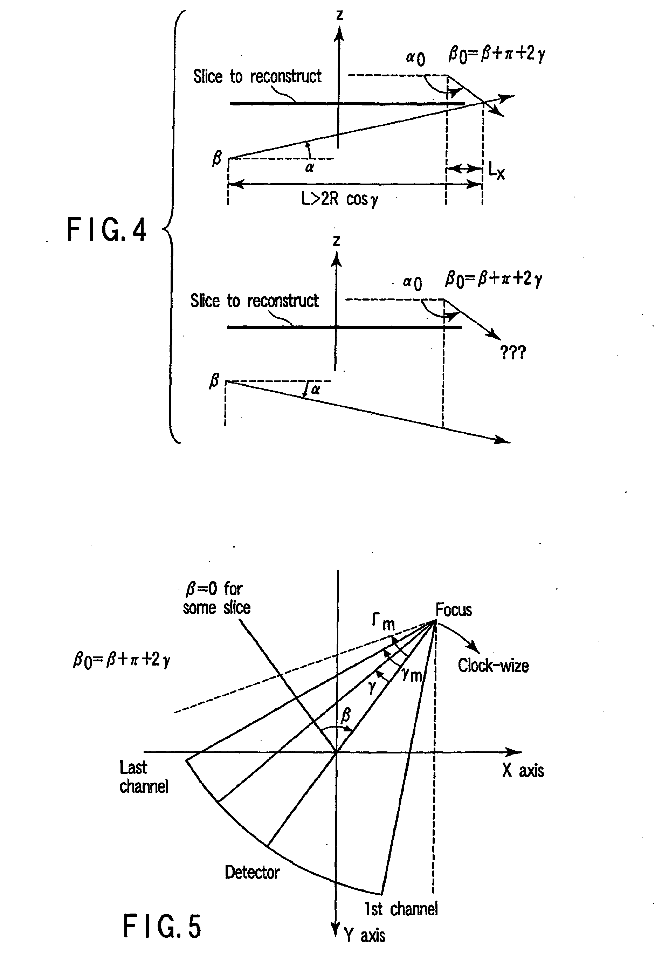

It is useful to restrict $\alpha_c$ as shown in Equation (B8) to avoid extraordinary cases, such as those shown in Fig. 4.

$$\alpha_c = \begin{cases} \alpha_0+\alpha_m & (|\alpha_c|>\alpha_0+\alpha_m) \\ \alpha_c & (otherwise) \end{cases} \tag{B8}$$

When the ray-sum of $\alpha$ does not pass through the slice of interest inside of the scan orbit, calculating $\alpha_c$ is meaningless and $\alpha_c$ can have a strange value. In this case, the MHS weight $w_o$ must be zero for such ray and $\alpha_c$ does not affect results at all. When $\beta= 0$ and $\alpha= 0$, $\alpha_c$ is unknown: one complementary ray can not be identified. However, it is not necessary to consider this case because in the current detector configuration, $\alpha$ cannot be 0. (The center of any detector row is not located at the mid-plane.) If the detector configuration is changed and there is a case for $\alpha = 0$, MHS may still be performed because the MHS weight for $\beta = 0$ any $\beta = 2\pi$ is 0.

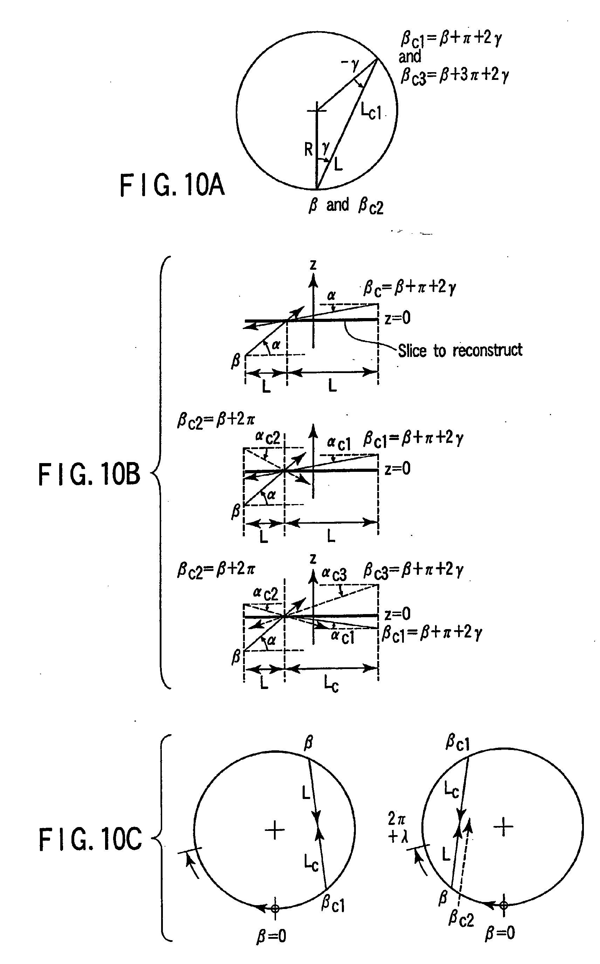

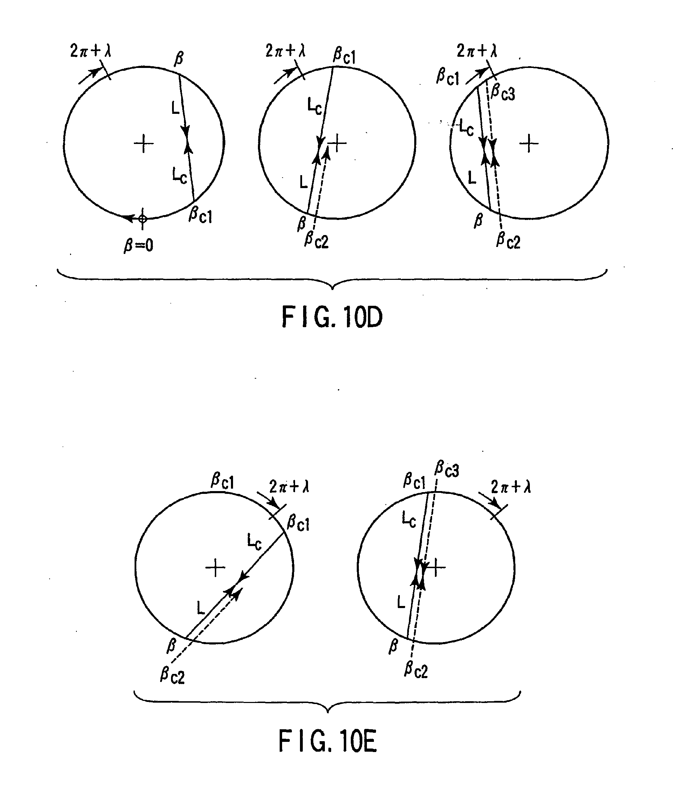

The complementary rays may be obtained when positions of focus and detector are exchanged (as shown in Fig. 10a) or at the same projection angle but in the different rotation (as shown in Fig. 10b). Let

$$\beta_h = (2\pi+\lambda)/2. \tag{C1}$$

There are two ways to obtain complementary rays: (1) using 6 complementary rays (the previous 3 rays and the following 3 rays) all the time without boundary conditions (the OS weight dismisses the non-existing rays automatically); or (2) using the "valid" complementary rays by considering the boundary conditions. The advantages of the former method include (1a) no need to bother with boundary conditions, and thus, (1b) simple coding. The advantages of the later method include (2a) a shorter processing time because all of the calculated rays are used (no waste rays).

The Method Using Six Complementary Rays All the Time

Projection angle and ray angle

$$\begin{cases}\beta_{c1} = \beta + \pi + 2\gamma \\ \gamma_{c1} = -\gamma\end{cases}, \qquad (C2)$$

$$\begin{cases}\beta_{c2} = \beta + 2\pi \\ \gamma_{c2} = \gamma\end{cases}, \qquad (C3)$$

$$\begin{cases}\beta_{c3} = \beta + 3\pi + 2\gamma = \beta_{c1} + 2\pi \\ \gamma_{c3} = -\gamma = \gamma_{c1}\end{cases}, \qquad (C4)$$

$$\begin{cases}\beta_{c(-1)} = \beta - \pi + 2\gamma = \beta_{c1} - 2\pi \\ \gamma_{c(-1)} = -\gamma = \gamma_{c1}\end{cases}, \qquad (C5)$$

$$\begin{cases}\beta_{c(-2)} = \beta - 2\pi \\ \gamma_{c(-2)} = \gamma\end{cases}, \qquad (C6)$$

$$\begin{cases}\beta_{c(-3)} = \beta - 3\pi + 2\gamma = \beta_{c(-1)} - 2\pi \\ \gamma_{c(-3)} = -\gamma = \gamma_{c(-1)}\end{cases}. \qquad (C7)$$

When $\beta = \beta_h$ and $\alpha = 0$, $\alpha_c$ is unknown: one complementary ray cannot be identified. However, it is not necessary to consider this case because in the current detector configuration, $\alpha$ cannot be 0. (The center of any detector row is not located at the mid-plane.) If the detector configuration is changed and there is a case for $a = 0$, the configuration will be still okay for MHS because the MHS weight for $\beta = 0$ and $\beta = 2\pi$ is 0. Additionally, in the OS case, it is possible to calculate two $\alpha_c$, one for $\beta = \beta_h - d\beta$ and the other for $\beta = \beta_h + d\beta$, and average them.

The complementary ray can be defined by using the following Equations 21 and 22.

The projection angle and the ray angle of n-complementary ray is defined by:

$$\beta_{c(n)} = \begin{cases}\beta + n\pi + 2\gamma & (n = odd) \\ \beta + 2n\pi & (n = even)\end{cases}, \qquad (21)$$

$$\gamma_{c(n)} = \begin{cases}-\gamma & (n = odd) \\ \gamma & (n = even)\end{cases}. \qquad (22)$$

The projected "in-plane" distances from the focus to the voxel of interest for primary and complementary ray are:

$$L = z_\beta / \tan\alpha, \qquad (23)$$

In order to avoid strange cases like $L > R + r_0$ (Fig. 4), L is clipped by:

$$L = \begin{cases}R + r_0/2 & (L > R + r_0/2) \\ R - r_0/2 & (L < R - r_0/2) \\ L & (otherwise)\end{cases}. \qquad (23a)$$

Subsequently,

$$L_{c(n)} = \begin{cases} 2R\cos\gamma - L & (n = odd) \\ L & (n = even) \end{cases}, \qquad (24)$$

$$z_\beta = -(\beta - \beta_0) \cdot H/2\pi, \qquad (25)$$

where $z_\beta$ denotes the z distance from each focus to the plane to reconstruct. The cone-angle for the complementary ray is:

$$\alpha_c = \tan^{-1}\left(z_{\beta(n)}/L_{c(n)}\right)$$
$$= \begin{cases} \tan^{-1} \dfrac{-(\beta + n\pi + 2\gamma - \beta_0)H}{2\pi(2R\cos\gamma - L)} & (n = odd) \\ \tan^{-1} \dfrac{-(\beta + 2n\pi - \beta_0)H}{2\pi L} & (n = even) \end{cases}. \qquad (26)$$

Again, restrict the angle for avoiding unnecessary cases. Specifically, Equation (26a) is used to calculate the cone-angle value. However, because the weighting curve shown in Fig. 8b is flat in regions where ($\alpha > \alpha_m$), clipping the cone-angle at $\alpha_m$ yields the same result.

In the equation, a is a cone angle at which the line connecting a specific ray of the cone beam and the focal point of the cone beam intersects with the plane perpendicular to the rotation axis (i.e., x axis), and $\alpha0$ is a cone angle for the ray applied to a detecting element near one end of any element-row of the detector array. The cone angle $\alpha0$ is not limited in particular. Therefore, it can be of the value for the ray applied to one end of the element-row. Nonetheless, as known in the art, the cone angle $\alpha0$ should better have an absolute value slightly smaller than the cone angle for the ray applied to one end of the element-column. The angle ( $\alpha0 + \alpha m$ ) is outside only with $\alpha m$ more than $\alpha0$. The channel corresponding to the angle ( $\alpha0 + \alpha m$ ) or $\alpha0$ may be an outermost one. Nevertheless, as is known in the art, too, it is desired that either outermost channel should lie between the lines corresponding to the cone angles $\alpha0$ and ( $\alpha0 + \alpha m$ ). A part of the data, which is not actually measured of the channels lying between the lines corresponding to the angles $\alpha0$ and ( $\alpha0 + \alpha m$ ), has been generated by extrapolation or is a copy of the data obtained at either outermost channel. The data obtained at some of the channels lying between the lines corresponding to the cone angles $\alpha0$ and ( $\alpha0 + \alpha m$ ) and the data obtained outside the line corresponding to the angle $\alpha m$, which have not been actually measured, are not so reliable. They are therefore weighted less than the actually measured data.

$$\alpha_c = \begin{cases} \alpha_m & (|\alpha_c| > \alpha_m) \\ \alpha_c & (otherwise) \end{cases}. \qquad (26a)$$

Note that when n = 0, Equations 21-26 give a primary ray.
Finally, the weights of overscan and half-scan are modified as shown in Equations 27 and 28 and used in Equation 3.

$$^{ConeOS}w_{\beta,\gamma,\alpha} = \frac{^{OS}w_\beta \cdot {}^{\overline{Cone}}w_{\beta,\gamma,\alpha}}{\sum_{n=-3}^{3}\left(^{OS}w_{\beta_c(n)} \cdot {}^{Cone}w_{\beta_c(n),\gamma_c(n),\alpha_c(n)}\right)}, \qquad (27)$$

and

$$^{ConeHS}w_{\beta,\gamma,\alpha} = \frac{^{HS}w_{\beta,\gamma} \cdot {}^{Cone}w_{\beta,\gamma,\alpha}}{\sum_{n=-1}^{1}\left(^{HS}w_{\beta_c(n),\gamma_c(n)} \cdot {}^{Cone}w_{\beta_c(n),\gamma_c(n),\alpha_c(n)}\right)}. \qquad (28)$$

The method described above has several advantages. First, the method of the present embodiment decreases weights to the invalid (extrapolated/replicated) rays and increases weight to the valid (measured) rays. Additionally, weights are normalized so that the redundancy of data is correctly compensated. Moreover, the method of the present embodiment will not change weights if all ray-sums are valid, all ray-sums are invalid, or there is no redundant data. Finally, the weight is a function of $\beta$, $\gamma$, and $\alpha$ in all helical pitches, and smoothly changes in any direction.

The cone angle dependent weight as described by $^{Cone}w$ (Equations (18-20)), may be arbitrary. It may include a sigmoid curve, an exponential, or the like. The weighting function, which is combined with $^{Cone}w$, may also be arbitrary. Even if cone angle dependent weight has been used, the method of the first embodiment is capable of enhancing performance by taking validity of each ray-sum into account. The reconstruction method of the present invention does not have to be "cone-beam," and may include such methods as parallel-fan-beam by rebinning (sorting) cone-beam data obtained at plural focus (cone vertexes) (like fan-to-parallel beam rebinning) and the like.

Additionally, according to a second aspect of the present invention, it is possible to modify the first embodiment to avoid giving too large a weight to the valid ray-sums.

When $wt_{max}$, has, for example, a value of 0.6, then

$$sw_{\beta,\gamma} = \sum_{n=-3}^{3} {}^{OS}w_{\beta_{c(n)}} \cdot {}^{Cone}w_{\beta_{c(n)},\gamma_{c(n)},\alpha_{c(n)}} \tag{29}$$

or

$$sw_{\beta,\gamma} = \sum_{n=-1}^{1} {}^{MHS}w_{\beta_{c(n)}} \cdot {}^{Cone}w_{\beta_{c(n)},\gamma_{c(n)},\alpha_{c(n)}}, \tag{30}$$

and

$$^{ConeOS}w_{\beta,\gamma,\alpha} = \begin{cases} wt_{max}, & \left({}^{Cone}w_{\beta,\gamma,\alpha} \cdot {}^{OS}w_{\beta,\gamma,\alpha} = w_m \text{ and } sw_{\beta,\gamma}/w_m \leq 1/wt_{max}\right) \\[2mm] \dfrac{{}^{Cone}w_{\beta,\gamma,\alpha} \cdot {}^{OS}w_{\beta,\gamma,\alpha} \times (1 - wt_{max})}{{}^{Cone}w_{\beta,\gamma,\alpha} \cdot {}^{OS}w_{\beta,\gamma,\alpha} + sw_{\beta,\gamma} - 1/2}, & \left({}^{Cone}w_{\beta,\gamma,\alpha} \cdot {}^{OS}w_{\beta,\gamma,\alpha} \neq w_m \text{ and } sw_{\beta,\gamma}/w_m \leq 1/wt_{max}\right) \\[2mm] \dfrac{{}^{Cone}w_{\beta,\gamma,\alpha} \cdot {}^{OS}w_{\beta,\gamma,\alpha}}{{}^{Cone}w_{\beta,\gamma,\alpha} \cdot {}^{OS}w_{\beta,\gamma,\alpha} + sw_{\beta,\gamma}}, & (otherwise) \end{cases}$$

$$\tag{31}$$

or

$$\text{ConeMHS}w_{\beta,\gamma,\alpha} = \begin{cases} wt_{max}, & \left(\text{Cone}w_{\beta,\gamma,\alpha}\cdot\text{MHS}w_{\beta,\gamma,\alpha} = w_m \text{ and } w_m \geq sw_{\beta,\gamma}\cdot wt_{max}\right) \\ \dfrac{\text{Cone}w_{\beta,\gamma,\alpha}\cdot\text{MHS}w_{\beta,\gamma,\alpha}\times(1-wt_{max})}{sw_{\beta,\gamma}-wt_{max}}, & \left(\text{Cone}w_{\beta,\gamma,\alpha}\cdot\text{MHS}w_{\beta,\gamma,\alpha} \neq w_m \text{ and } w_m \geq sw_{\beta,\gamma}\cdot wt_{max}\right) \\ \dfrac{\text{Cone}w_{\beta,\gamma,\alpha}\cdot\text{MHS}w_{\beta,\gamma,\alpha}}{sw_{\beta,\gamma}}, & \left(otherwise\right) \end{cases}$$

$$(32)$$

where $w_m$ is the maximum weight among all the primary and the complementary rays

$$\left(^{OS}w_{\beta_c(n)}\cdot^{Cone}w_{\beta_c(n),\gamma_c(n),\alpha_c(n)} \text{ or } ^{MHS}w_{\beta_c(n)}\cdot^{Cone}w_{\beta_c(n),\gamma_c(n),\alpha_c(n)}\right). \qquad (33)$$

The method according to the above-described second aspect of the present invention provides decreased image noise. The noise is most effectively reduced when data are weighted equally. The first embodiment sometimes results in disproportionate weighting of the data, which causes increased image noise. By capping the value at $\alpha_m$, it is possible to achieve a better balancing of the validity of the data, the ability to reduce image noise, and the image quality.

Additionally, according to a third aspect of the present invention, it is possible to obtain a more efficient method of data weighting. The third embodiment method does not search non-existing ray-sums. For example, in a case of MHS weighting, the projection data ranges for $2\pi$. Therefore, it is possible to use Equation (34) instead of Equation (21), thereby reducing the number of complementary ray-sums to calculate from 3 to 2.

$$\beta_{c(n=1)} = \begin{cases} \beta+\pi+2\gamma & (\beta'+2\gamma \leq \pi) \\ \beta-\pi+2\gamma & (otherwise) \end{cases}. \qquad (34)$$

The Method Using Six Complementary Rays All the Time in OS

$$wt_{\alpha,\beta,\gamma} = \frac{w_r(\alpha)\cdot w_{o,\beta}}{w_r(\alpha)\cdot w_{o,\beta} + \displaystyle\sum_{n=-3}^{-1} w_r(\alpha_{c(n)})\cdot w_{oc(n),\beta c(n),\gamma(n)} + \sum_{n=1}^{3} w_r(\alpha_{cn})\cdot w_{ocn,\beta cn,\gamma n}}, \qquad (C1)$$

where

$$x(\alpha) = \begin{cases} 0 & |\alpha| \leq \alpha_0 \\ (|\alpha|-\alpha_0)/\alpha_m & \alpha_0 < |\alpha| \leq \alpha_0+\alpha_m \\ 1 & \alpha_0+\alpha_m < |\alpha| \end{cases}, \qquad (C2)$$

and

$$w_r = 1-a\cdot(3x^2-2x^3). \qquad (C3)$$

Note: 0 < a < 1. When a = 0, Eq. (C1) provides OS weight, $wt_{\alpha,\beta,\gamma} = w_{o,\beta}$.

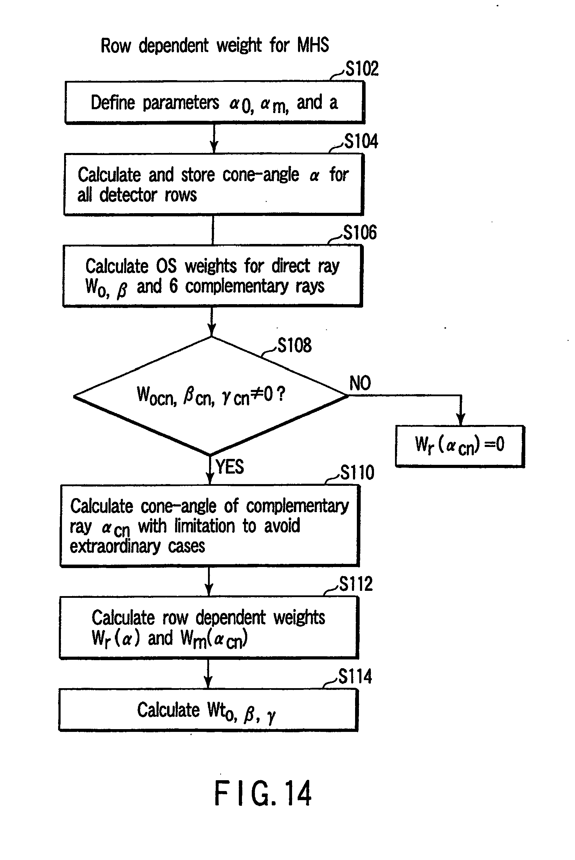

As illustrated in Fig. 14, the method for using six complementary rays all the time includes several steps. The method begins with step S102: Define parameters $\alpha_0$, $\alpha_m$, and a. Step S104: Calculate and store cone-angle $\alpha$ for all of detector rows. Step S106: Calculate OS weights for direct ray $w_{o,\beta}$ and 6 complementary rays $w_{ocn,\beta cn,\gamma cn}$. Step S108: If $w_{ocn,\beta cn,\gamma cn} \neq 0$, do steps S 110 and S 112. Else if $w_{ocn,\beta cn,\gamma cn} = 0$, let $w_r(\alpha_{cn}) = 0$. Step S110: Calculate cone-angle of complementary ray, $\alpha_{cn}$, with limitation for avoiding extraordinary cases. Step S 112: Calculate row dependent weights $w_r(\alpha)$ and $w_{rm}(\alpha_{cn})$. Step S114: Calculate $wt_{o,\beta,\gamma}$ by Equation (C1), shown above.

The Method to Use Valid Complementary Rays Only in OS

A similar approach can be taken for OS or other weighting methods to reduce the number of ray-sums from 7 to 4. First, the weight is applied. Then, the two weights for the direct ray and the complementary ray are normalized.

$$wt_{\alpha,\beta,\gamma} = \frac{w_r(\alpha) \cdot w_{o,\beta}}{w_r(\alpha) \cdot w_{o,\beta} + \sum_{n=1}^{3} w_r(\alpha_{cn}) \cdot w_{ocn,\beta cn,\gamma n}}. \qquad \text{(C1)'}$$

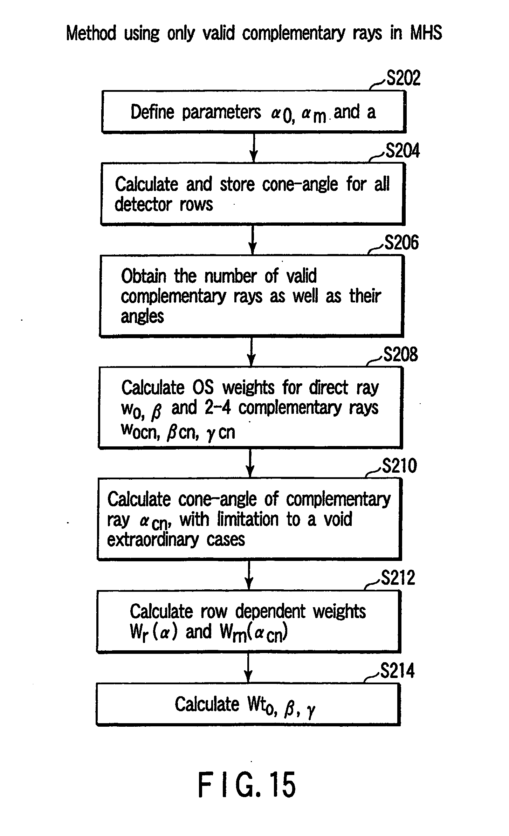

The method for using valid complementary rays only is shown in Fig. 15. The method includes several steps, beginning with step S202: Define parameters $\alpha_0$, $\alpha_m$, and a. Step S204: Calculate and store cone-angle $\alpha$ for all of detector rows. Step S206: Obtain the number of valid complementary rays as well as their angles. Step S208: Calculate OS weights for direct ray $w_{o,\beta}$ and 2-4 complementary rays $w_{ocn,\beta cn,\gamma cn}$. Step S210: Calculate cone-angle of complementary ray, $\alpha_{cn}$, with limitation for avoiding extraordinary cases. Step S212: Calculate row dependent weights $w_r(\alpha)$ and $w_{in}(\alpha_{cn})$. Step S214: Calculate $wt_{o,\beta,\gamma}$ by Equation (C1)'.

An example of parameters is shown below:

$$\alpha_0 = \tan^{-1} \frac{d \cdot \left( \frac{Nrow-1}{2} + r1 \right)}{R}, \qquad \text{(D1)}$$

$$\alpha_m = \tan^{-1} \frac{d \cdot \left( \frac{Nrow-1}{2} + r2 \right)}{R} - \alpha_0, \qquad \text{(D2)}$$

$$a = 0.9. \qquad \text{(D3)}$$

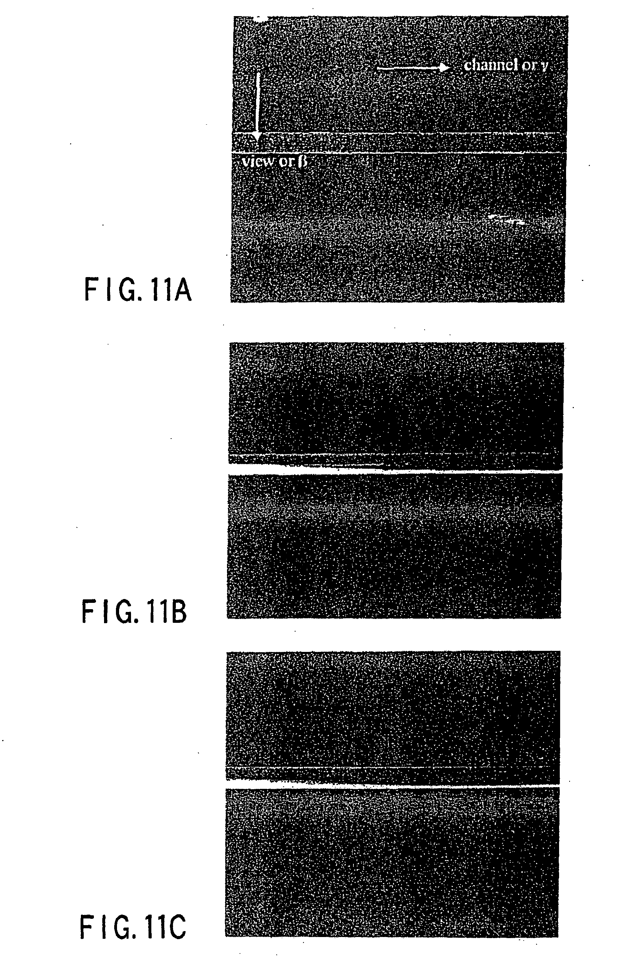

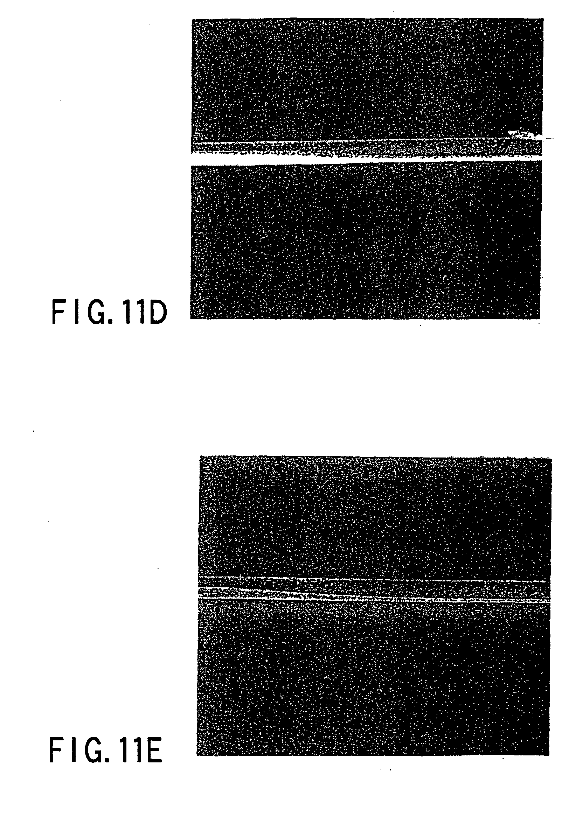

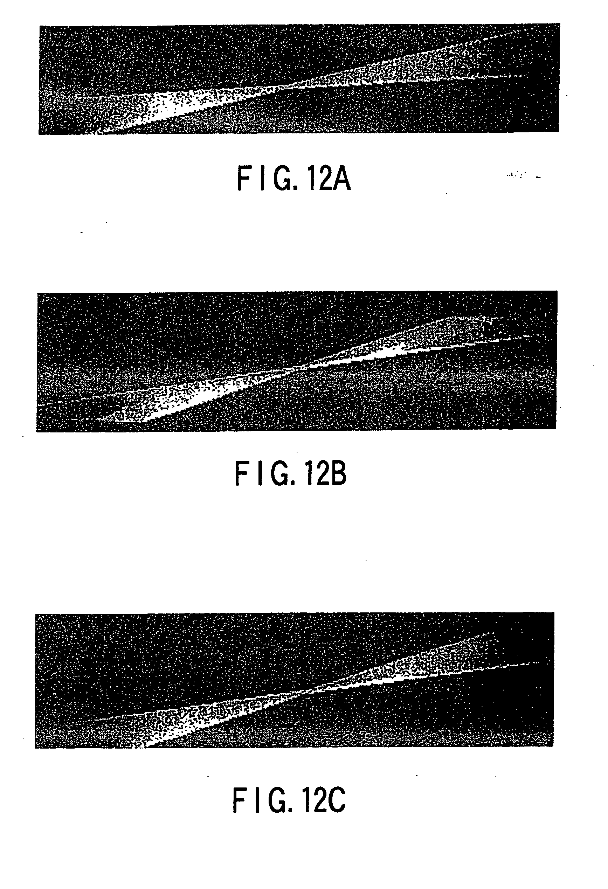

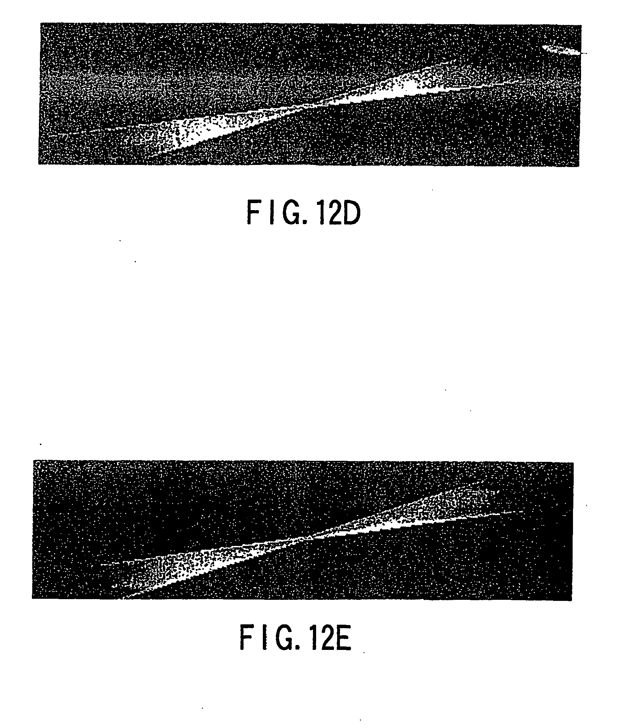

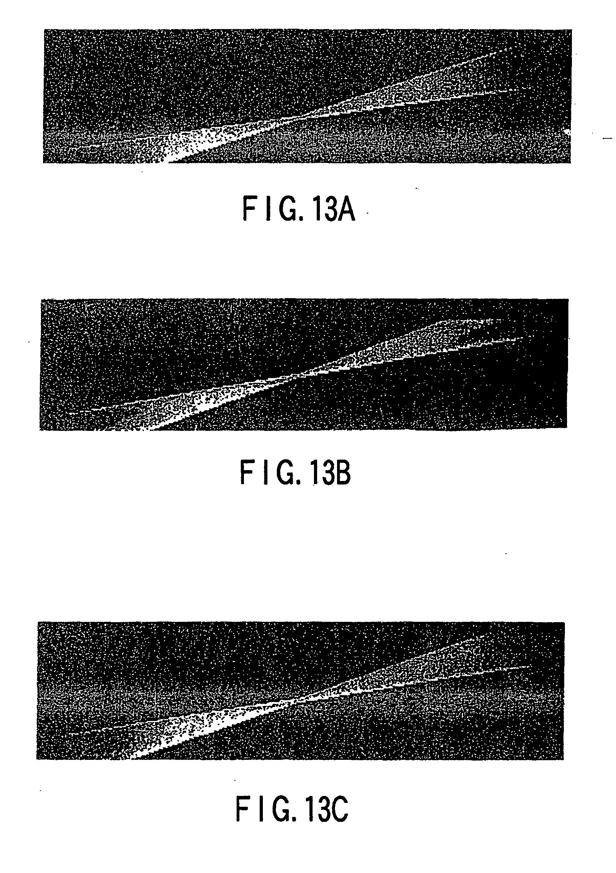

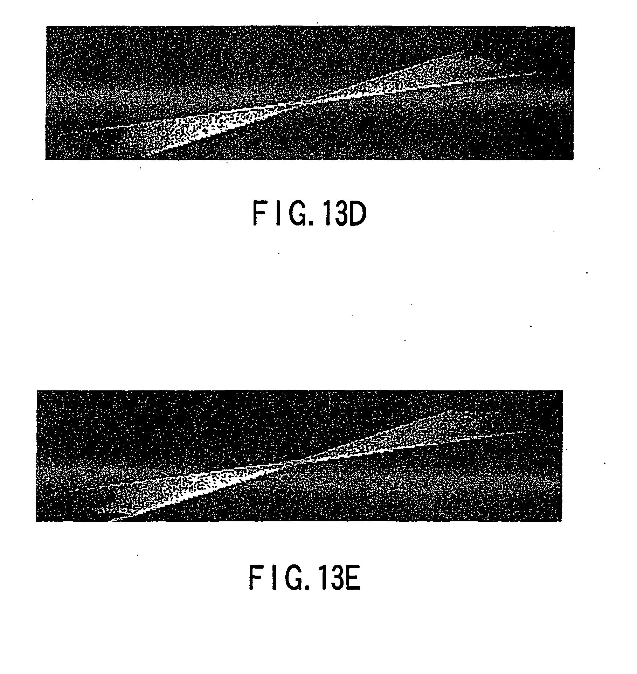

Examples of row-OS weight are shown in Figs. 11-13. Note that these Figs. only show detector rows used in reconstruction. Fig. 11b: When (r2-r1) is small, we observe rapid change in view direction as well as whip-type pattern. When (r2-r1) is large, this change and whip is feathered (Fig. 11c). When r1 < 0, there is no whip and rapid change (Fig. 11d). Fig. 12b: When (r2-r1) is small, the change in row direction is also rapid. It is smoothed when (r2-r1) is large (Fig. 12c). Fig. 12d: (r1 < 0) can be an option, but may reduce photon utilization rate, that is, increase image noise. Fig. 12e: small a may not be able to reduce the effect of extrapolation. Fig. 13: The center channel shows symmetric weighting, while (Fig. 12) shows asymmetric weight.

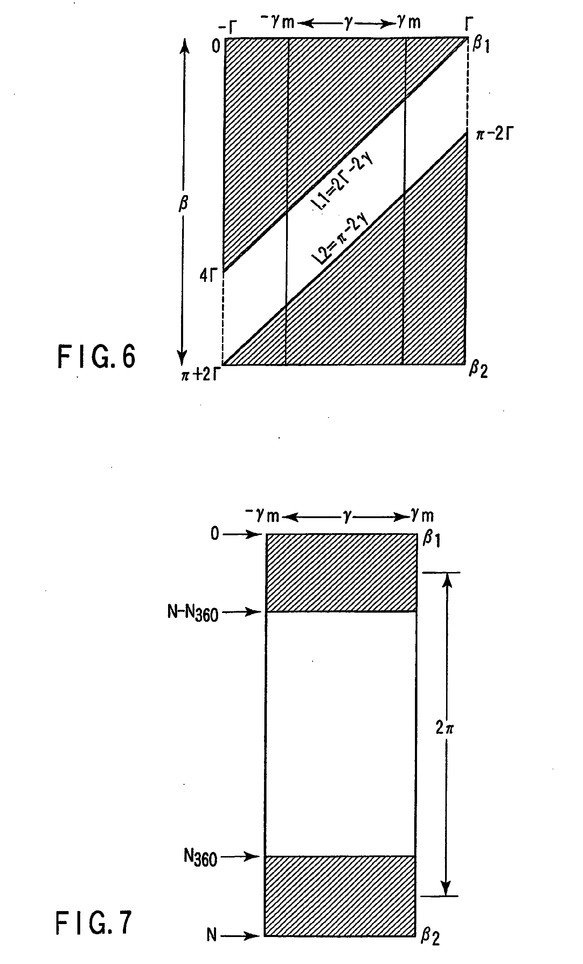

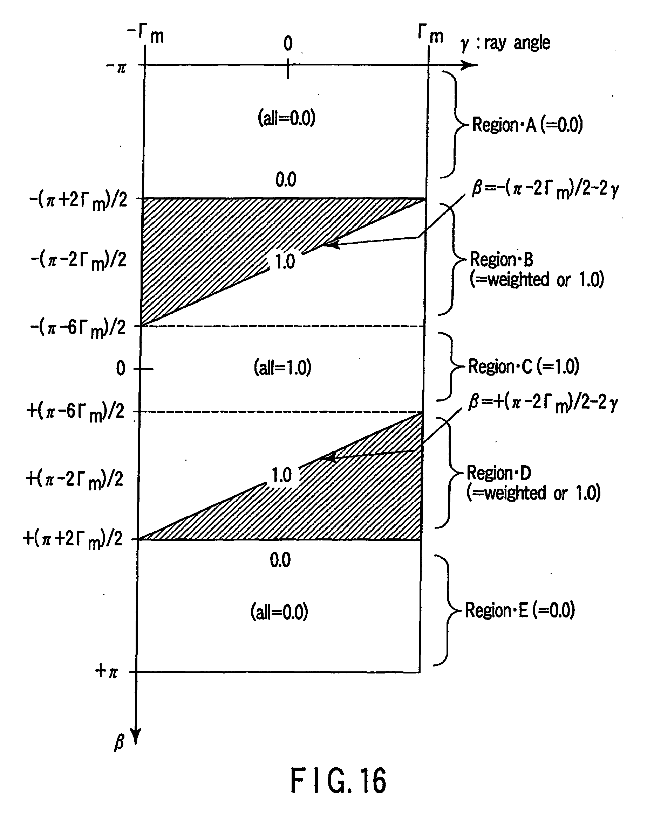

The first formula applied is for MHS weight $w_{p,\beta,\gamma}$ (independent of cone angle $\alpha$), as illustrated in Fig. 16, which is an alternative depiction of the MHS weight of Fig. 6. Below is a modified MHS weight when $\beta = [- (\pi + 2\Gamma_m)/2, (\pi + 2\Gamma_m)/2]$, rather than when $\beta = [0, \pi + 2\Gamma_m]$. It is also possible to use a version for $\beta = [0, \pi + 2\Gamma_m]$. As shown in Fig. 16, if $(\beta < -(\pi + 2\Gamma_m)/2$ then Region A applies and

$$w_{p,\beta,\gamma} = 0 \qquad \text{(A1)}$$

Else if $(-(\pi+2\Gamma_m)/2 \leq \beta < -(\pi-6\Gamma_m)/2$ Region B applies.

If $\left( \gamma < \left( -\frac{\pi}{2} - \Gamma_m - \beta \right) \middle/ 2 \right.$, then the left side region: reversed triangle area applies.

$$x_{p,\beta,\gamma} = \left(\frac{\pi}{2} + \Gamma_m + \beta\right)\Big/2(\Gamma_m - \gamma) \qquad (A2)$$

$$w_{p,\beta,\gamma} = 3x_{p,\beta,\gamma}^2 - 2x_{p,\beta,\gamma}^3 \qquad (A3)$$

Otherwise if $((\pi/2 - \Gamma_m-\beta)/2 \le \gamma)$, then the right side region: triangle (exists when $\Gamma_m > \pi/2-2\gamma_m$) applies and

$$x_{p,\beta,\gamma} = \left(\frac{\pi}{2} + \Gamma_m - \beta\right)\Big/2(\Gamma_m + \gamma) \qquad (A4)$$

$$w_{p,\beta,\gamma} = 3x_{p,\beta,\gamma}^2 - 2x_{p,\beta,\gamma}^3 \qquad (A5)$$

Else the center region: flat applies and

$$w_{p,\beta,\gamma} = 1 . \qquad (A6)$$

Else if $(- (\pi- 6\Gamma_m)2 \le \beta < (\pi - 6\Gamma_m)/2$ then Region C applies and

$$w_{p,\beta,\gamma} = 1 \qquad (A7)$$

Else if $((\pi-6\Gamma_m)/< (\pi+2\Gamma_m)/2)$Region D applies and the left side of Region D is taken care of by Region B.

If $\left(\left(\frac{\pi}{2} - \Gamma_m - \beta\right)\Big/2 \le \chi\right.$ then the right side region, triangle applies:

$$x_{p,\beta,\gamma} = \left(\frac{\pi}{2} + \Gamma_m - \beta\right)\Big/2(\Gamma_m + \gamma) \qquad (A8)$$

$$w_{p,\beta,\gamma} = 3x_{p,\beta,\gamma}^2 - 2x_{p,\beta,\gamma}^3 \qquad (A9)$$

Else in the center region, flat:

$$w_{p,\beta,\gamma} = 1 \qquad (A10)$$

Else if $((\pi + 2\Gamma_m)/2 \le \beta$ Region E applies:

$$w_{p,\beta,\gamma} = 0 \qquad (A11)$$

Obviously, numerous modifications and variations of the present invention are possible in light of the above teachings. It is therefore to be understood that within the scope of the appended claims, the invention may be practiced otherwise than as specifically described herein.

**Claims**

1. An X-ray CT apparatus, **characterized by** comprising:

a helical scanning device configured to collect projection data while at least one of a gantry (1) and a couch (6) moves along an axial direction of the couch (6), the helical scanning device including,
an X-ray source (3) configured to generate X-rays, and
a detector (5) having detector elements (5A) arranged in a plurality of rows along the axial direction and configured to produce the projection data; and
a processor (12) comprising,
a weighted device configured to apply a weighting function including
a cone-angle dependent weight to the projection data, thereby obtaining weighted data,
a filtering device configured to filter the weighted data, and
a backprojecting device configured to backproject the weighted data taking cone-angle into account,
the apparatus being configured such that projection data whose cone angle is outside a predetermined cone angle ($\alpha 0$) is not measured, the non-measured projection data is generated by extrapolation or copying and the generated projection data is weighted according to the cone angle.

2. The apparatus according to claim 1, **characterized in that** the backprojecting device is configured to perform Feldkamp reconstruction.

3. The apparatus according to claim 1, **characterized in that** the weighting function includes an MHS function.

4. The apparatus according to claim 1, **characterized in that** the weighting function includes an OS function.

5. A method for processing data obtained from a CT scan, said CT scan being performed by an X-ray CT apparatus, comprising:

a helical scanning device configured to collect projection data while at least one of a gantry (1) and a couch (6) moves along an axial direction of the couch (6), the helical scanning device including,
an X-ray source (3) configured to generate X-rays, and
a detector (5) having detector elements (5A) arranged in a plurality of rows along the axial direction and configured to produce the projection data,
said method comprising:

applying a weighting function including a cone-angle dependent weight to the projection data, thereby obtaining weighted data,
filtering the weighted data, and
backprojecting the weighted data taking cone-angle into account,

wherein the projection data whose cone angle is outside a predetermined cone angle ($\alpha 0$) is not measured, the non-measured projection data is generated by extrapolation or copying and the generated projection data is weighted according to the cone angle.

6. The method of claim 5, further comprising calculating at least one OS weight for at least one direct ray and a plurality of complementary rays; determining a value of the at least one OS weight; and calculating a second cone-angle for the plurality of complementary rays wherein calculating the second cone-angle is performed with a limitation to avoid extraordinary cases.

7. The method of claim 5, further comprising calculating at least one OS weight for at least one direct ray and a plurality of complementary ray; and determining a value of the at least one OS weight wherein a a number of valid complementary rays is obtained before determining a value of the at least one OS weight.

8. The method of claim 7, **characterized in that** a number of the plurality of complementary rays is two or four.

9. The method of claim 7, **characterized in that** a number of the plurality of complementary ray is six or more.

10. A computer program product storing instructions for execution on a computer system, which when executed by the computer system, causes the computer system to perform the method recited in any one of claims 5 to 9.

**Patentansprüche**

1. Röntgen-CT Vorrichtung **gekennzeichnet durch**
   eine helisch abtastende Vorrichtung, die konfiguriert ist zum Sammeln von Projektionsdaten während sich ein Ständer (1) und/oder eine Liege (6) entlang einer Axialrichtung der Liege (6) bewegt, wobei die helisch abtastende Vorrichtung aufweist:

   eine Röntgenstrahlquelle (3), die konfiguriert ist zum Erzeugen von Röntgenstrahlen;
   einen Detektor (5) mit Detektorelementen (5A), die in einer Mehrzahl von Reihen entlang der Axialrichtung angeordnet und konfiguriert sind zum Erzeugen der Projektionsdaten; und
   einen Prozessor (12) enthaltend
   eine Gewichtungsvorrichtung, die konfiguriert ist zum Anwenden einer Gewichtungsfunktion enthaltend eine kegelwinkelabhängige Gewichtung der Projektionsdaten, wodurch gewichtete Daten gewonnen werden,
   eine Filtervorrichtung, die konfiguriert ist zum Filtern der gewichteten Daten, und
   eine Rückprojektionsvorrichtung, die konfiguriert ist zur Rückprojektion der gewichteten Daten unter Berücksichtigung eines Kegelwinkels,

   wobei die Vorrichtung derart konfiguriert ist, dass Projektionsdaten, deren Kegelwinkel außerhalb eines vorbestimmten Kegelwinkels ($\alpha$0) ist, nicht gemessen wird, die nicht gemessenen Projektionsdaten **durch** Extrapolation oder Kopieren erzeugt werden, und die erzeugten Projektionsdaten gemäß dem Kegelwinkel gewichtet werden.

2. Vorrichtung nach Anspruch 1, **dadurch gekennzeichnet, dass** die Rückprojektionsvorrichtung konfiguriert ist zur Durchführung einer Feldkamp-Rekonstruktion.

3. Vorrichtung nach Anspruch 1, **dadurch gekennzeichnet, dass** die Gewichtungsfunktion eine MHS-Funktion enthält.

4. Vorrichtung nach Anspruch 1, **dadurch gekennzeichnet, dass** die Gewichtungsfunktion eine OS-Funktion enthält.

5. Verfahren zum Verarbeiten von Daten, die gewonnen werden aus einer CT-Abtastung, wobei die CT-Abtastung von einer Röntgen-CT Vorrichtung durchgeführt wird, die
   eine helisch abtastende Vorrichtung enthält, die konfiguriert ist zum Sammeln von Projektionsdaten während ein Ständer (1) und/oder eine Liege (6) sich entlang einer Axialrichtung der Liege (6) bewegt, wobei die helisch abtastende Vorrichtung aufweist
   eine Röntgenstrahlquelle (3), die konfiguriert ist zum Erzeugen von Röntgenstrahlen, und
   einen Detektor (5) mit Detektorelementen (5A), die in einer Mehrzahl von Reihen entlang der Axialrichtung angeordnet und konfiguriert sind zum Erzeugen von Projektionsdaten, wobei das Verfahren aufweist:

   Anwenden einer Gewichtungsfunktion mit einer kegelwinkelabhängigen Gewichtung auf die Projektionsdaten, wodurch gewichtete Daten gewonnen werden,
   Filtern der gewichteten Daten, und
   Rückprojizieren der gewichteten Daten unter Berücksichtigung des Kegelwinkels,

   wobei Projektionsdaten, deren Kegelwinkel außerhalb eines vorbestimmten Kegelwinkels ($\alpha$0) liegt, nicht gemessen werden, die nicht gemessenen Projektionsdaten durch Extrapolation oder Kopieren erzeugt werden, und die erzeugten Projektionsdaten gemäß dem Kegelwinkel gewichtet werden.

6. Verfahren nach Anspruch 5, ferner mit einem Berechnen mindestens einer OS-Gewichtung für mindestens einen Direktstrahl und eine Mehrzahl von Komplementärstrahlen; Bestimmen eines Werts der mindestens einen OS-Gewichtung; und Berechnen eines zweiten Kegelwinkels für die Mehrzahl der Komplementärstrahlen, wobei das Berechnen des zweiten Kegelwinkels durchgeführt wird mit einer Einschränkung, um außerplanmäßige Fälle zu vermeiden.

7. Verfahren nach Anspruch 5, ferner mit einem Berechnen mindestens einer OS-Gewichtung für mindestens einen Direktstrahl und eine Mehrzahl von Komplementärstrahlen; und Bestimmen eines Werts der mindestens einen OS-Gewichtung, wobei eine Anzahl von gültigen Komplementärstrahlen gewonnen wird bevor ein Wert der mindestens einen OS-Gewichtung bestimmt wird.

8. Verfahren nach Anspruch 7, **dadurch gekennzeichnet, dass** eine Anzahl von der Mehrzahl von Komplementärstrahlen 2 oder 4 ist.

9. Verfahren nach Anspruch 7, **dadurch gekennzeichnet, dass** eine Anzahl der Mehrzahl von Komplementärstrahlen 6 oder größer ist.

10. Computerprogrammprodukt, das Anweisungen speichert zur Ausführung auf einem Computersystem, die, wenn sie von dem Computersystem ausgeführt werden, das Computersystem veranlassen zum Durchführen des Verfahrens gemäß einem der Ansprüche 5 bis 9.

**Revendications**

1. Appareil CT rayons X, **caractérisé en ce qu'**il comprend :

un dispositif de scannage en hélice configuré pour collecter des données de projection tandis qu'au moins un élément pris parmi un portique (1) et une couchette (6) se déplace suivant une direction axiale de la couchette (6), le dispositif de scannage en hélice incluant :

une source de rayons X (3) configurée pour générer des rayons X ; et
un détecteur (5) comportant des éléments de détecteur (5A) agencés selon une pluralité de rangées suivant la direction axiale et configurés pour produire les données de projection ; et
un processeur (12) comprenant :

un dispositif de pondération configuré pour appliquer une fonction de pondération incluant un poids dépendant de l'angle de cône sur les données de projection, d'où ainsi l'obtention de données pondérées ;
un dispositif de filtrage configuré pour filtrer les données pondérées ; et
un dispositif de rétroprojection configuré pour rétroprojeter les données pondérées en prenant en compte l'angle de cône,
l'appareil étant configuré de telle sorte que des données de projection dont l'angle de cône est à l'extérieur d'un angle de cône prédéterminé ($\alpha 0$) ne soient pas mesurées, que les données de projection non mesurées soient générées par extrapolation ou copie et que les données de projection générées soient pondérées conformément à l'angle de cône.

2. Appareil selon la revendication 1, **caractérisé en ce que** le dispositif de rétroprojection est configuré pour réaliser une reconstruction de Feldkamp.

3. Appareil selon la revendication 1, **caractérisé en ce que** la fonction de pondération inclut une fonction MHS.

4. Appareil selon la revendication 1, **caractérisé en ce que** la fonction de pondération inclut une fonction OS.

5. Procédé pour traiter des données obtenues à partir d'un scan CT, ledit scan CT étant réalisé au moyen d'un appareil CT rayons X, comprenant :

un dispositif de scannage en hélice configuré pour collecter des données de projection tandis qu'au moins un élément pris parmi un portique (1) et une couchette (6) se déplace suivant une direction axiale de la couchette (6), le dispositif de scannage en hélice incluant :

une source de rayons X (3) configurée pour générer des rayons X ; et
un détecteur (5) comportant des éléments de détecteur (5A) agencés selon une pluralité de rangées suivant la direction axiale et configurés pour produire les données de projection,
ledit procédé comprenant :

l'application d'une fonction de pondération incluant un poids dépendant d'un angle de cône sur les données de projection, d'où ainsi l'obtention de données pondérées ;
le filtrage des données pondérées ; et
la rétroprojection des données pondérées en prenant en compte l'angle de cône,

dans lequel des données de projection dont l'angle de cône est à l'extérieur d'un angle de cône prédéterminé ($\alpha 0$) ne sont pas mesurées, les données de projection non mesurées sont générées par extrapolation ou copie et les données de projection générées sont pondérées conformément à l'angle de cône.

6. Procédé selon la revendication 5, comprenant en outre le calcul d'au moins un poids OS pour au moins un rayon direct et une pluralité de rayons complémentaires ; la détermination d'une valeur de l'au moins un poids OS ; et le calcul d'un second angle de cône pour la pluralité de rayons complémentaires où le calcul du second angle de cône est réalisé avec une limitation pour éviter des cas extraordinaires.

7. Procédé selon la revendication 5, comprenant en outre le calcul d'au moins un poids OS pour au moins un rayon direct et une pluralité de rayons complémentaires ; et la détermination d'une valeur de l'au moins un poids OS où un certain nombre de rayons complémentaires valides sont obtenus avant la détermination d'une valeur de l'au moins un poids OS.

8. Procédé selon la revendication 7, **caractérisé en ce que** le nombre de la pluralité de rayons complémentaires est de deux ou quatre.

9. Procédé selon la revendication 7, **caractérisé en ce que** le nombre de la pluralité de rayons complémentaires est de six ou plus.

10. Produit de programme d'ordinateur qui stocke des instructions pour une exécution sur un système d'ordinateur, lequel produit de programme, lorsqu'il est exécuté par le système d'ordinateur, a pour effet que le système d'ordinateur réalise le procédé revendiqué selon l'une quelconque des revendications 5 à 9.

FIG. 1A

FIG. 1B

FIG. 1C

**FIG. 2**

**FIG. 3A**

**FIG. 3B**

FIG.4

z

Slice to reconstruct

$\alpha_0$   $\beta_0 = \beta + \pi + 2\gamma$

$\beta$   $\alpha$

$L_x$

$L > 2R\cos\gamma$

z

Slice to reconstruct

$\alpha_0$   $\beta_0 = \beta + \pi + 2\gamma$

???

$\beta$   $\alpha$

$\beta = 0$ for
some slice

$\beta_0 = \beta + \pi + 2\gamma$

$\Gamma_m$   Focus

Clock-wize

$\gamma_m$

$\gamma$

$\beta$

Last
channel

X axis

Detector

1st channel

FIG.5   Y axis

FIG.6

FIG.7

FIG. 8A

FIG. 8B

FIG. 9

FIG. 10A

FIG. 10B

FIG. 10C

FIG. 10D

FIG. 10E

FIG. 11A

FIG. 11B

FIG. 11C

FIG. 11D

FIG. 11E

F I G. 12A

F I G. 12B

F I G. 12C

F I G. 12D

F I G. 12E

F I G. 13A

F I G. 13B

F I G. 13C

F I G. 13D

F I G. 13E

Row dependent weight for MHS

```
                                                    S102
┌─────────────────────────────────────┐
│ Define parameters α0, αm, and a      │
└─────────────────────────────────────┘
                    │
                    ▼                               S104
┌─────────────────────────────────────┐
│ Calculate and store cone-angle α for │
│ all detector rows                    │
└─────────────────────────────────────┘
                    │
                    ▼                               S106
┌─────────────────────────────────────┐
│ Calculate OS weights for direct ray  │
│ W0, β and 6 complementary rays       │
└─────────────────────────────────────┘
                    │
                    ▼                     S108
              ╱─────────────╲                      NO
            ╱                 ╲
          ╱  Wocn, βcn, γcn≠0 ? ╲──────────────┐
            ╲                 ╱                 │
              ╲─────────────╱                   ▼
                    │ YES                ┌──────────────┐
                    ▼           S110     │ Wr(αcn) =0   │
┌─────────────────────────────────────┐ └──────────────┘
│ Calculate cone-angle of complementary│
│ ray αcn with limitation to avoid     │
│ extraordinary cases                  │
└─────────────────────────────────────┘
                    │
                    ▼                               S112
┌─────────────────────────────────────┐
│ Calculate row dependent weights      │
│ Wr(α) and Wm(αcn)                    │
└─────────────────────────────────────┘
                    │
                    ▼                               S114
┌─────────────────────────────────────┐
│ Calculate Wt0, β, γ                  │
└─────────────────────────────────────┘
```

FIG. 14

Method using only valid complementary rays in MHS

```
                                                    ┌─S202
        ┌──────────────────────────────────────┐
        │  Define parameters $\alpha_0$, $\alpha_m$ and a  │
        └──────────────────────────────────────┘
                         │
                         ▼                           ┌─S204
        ┌──────────────────────────────────────┐
        │  Calculate and store cone-angle for all │
        │  detector rows                          │
        └──────────────────────────────────────┘
                         │
                         ▼                           ┌─S206
        ┌──────────────────────────────────────┐
        │  Obtain the number of valid             │
        │  complementary rays as well as their    │
        │  angles                                 │
        └──────────────────────────────────────┘
                         │
                         ▼                           ┌─S208
        ┌──────────────────────────────────────┐
        │  Calculate OS weights for direct ray    │
        │  $W_0$, $\beta$ and 2-4 complementary rays │
        │  $W_{ocn}$, $\beta_{cn}$, $\gamma_{cn}$ │
        └──────────────────────────────────────┘
                         │
                         ▼                           ┌─S210
        ┌──────────────────────────────────────┐
        │  Calculate cone-angle of complementary  │
        │  ray $\alpha_{cn}$, with limitation to a void │
        │  extraordinary cases                    │
        └──────────────────────────────────────┘
                         │
                         ▼                           ┌─S212
        ┌──────────────────────────────────────┐
        │  Calculate row dependent weights        │
        │  $W_r(\alpha)$ and $W_m(\alpha_{cn})$   │
        └──────────────────────────────────────┘
                         │
                         ▼                           ┌─S214
        ┌──────────────────────────────────────┐
        │  Calculate $Wt_0$, $\beta$, $\gamma$    │
        └──────────────────────────────────────┘
```

# F I G. 15

$-\Gamma_m$     0     $\Gamma_m$    $\gamma$ : ray angle

$-\pi$

(all=0.0)      Region·A (=0.0)

0.0

$-(\pi+2\Gamma_m)/2$      $\beta = -(\pi-2\Gamma_m)/2-2\gamma$

$-(\pi-2\Gamma_m)/2$    1.0      Region·B (=weighted or 1.0)

$-(\pi-6\Gamma_m)/2$

0    (all=1.0)      Region·C (=1.0)

$+(\pi-6\Gamma_m)/2$      $\beta = +(\pi-2\Gamma_m)/2-2\gamma$

$+(\pi-2\Gamma_m)/2$    1.0      Region·D (=weighted or 1.0)

$+(\pi+2\Gamma_m)/2$

0.0

(all=0.0)      Region·E (=0.0)

$+\pi$

$\beta$

# F I G. 16

FIG. 17A

FIG. 17B

FIG. 17C

F I G. 18A

F I G. 18B

F I G. 18C

FIG. 18D

FIG. 18E

F I G. 19A

F I G. 19B

F I G. 19C

FIG. 19D

FIG. 19E

FIG. 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2825352 B
- US 5825842 A
- US 6408042 B
- US 5430783 A, Hu

### Non-patent literature cited in the description

- **Y. SAITO ; H. ARADATE ; H. MIYAZAKI ; K. IGARASHI ; H. IDE.** Development of a large area 2-dimensional detector for real-time 3-dimensional CT (4D-CT. *Radiology,* 2000, vol. 217, 405
- **Y. SAITO ; H. ARADATE ; H. MIYAZAKI ; K. IGARASHI ; H. IDE.** Large area two-dimensional detector system for real-time three-dimensional CT (4D-CT. *Proc. of SPIE Med. Imag. Conf.,* 2001, vol. 4320, 775-782
- **L. A. FELDKAMP ; L. C. DAVIS ; J. W. KRESS.** Practical cone-beam algorithm. *J. Opt. Soc. Am. A,* 1984, vol. 6, 612-19
- **L. G. ZENG ; G. T. GULLBERG.** Short-scan cone beam algorithm for circular and noncircular detector orbit. *Proc. of SPIE Med. Imag. Conf,* 1990, vol. 1233, 453-463
- **H. KUDO ; T. SAITO.** Three-dimensional helical-scan computed tomography using cone-beam projections. *J. Electron. Information Commun. Soc. Japan, J74-D-II,* 1991, 1108-1114
- **G. WANG ; T. H. LIN ; P. C. CHENG ; D. M. SHI-NOZAKI.** A general cone-beam reconstruction algorithm. *IEEE Trans. Med. Imaging,* 1993, vol. 12, 486-496
- **K. WIESENT ; K. BARTH ; N. NOVAB et al.** Enhanced 3-D-reconstruction algorithm for C-arm systems suitable for interventional procedures. *IEEE Trans. Med. Imaging,* 2000, vol. 19, 391-403
- **M. D. SILVER ; K. TAGUCHI ; K. S: HAN.** Field-of-view dependent helical pitch in multi-slice CT. *Proc. of SPIE Med. Imag. Conf.,* 2001, vol. 4320, 839-850
- **M. D. SILVER ; K. TAGUCHI ; I. A. HEIN.** A simple algorithm for increased helical pitch in cone-beam CT. *The Sixth International Meeting on Fully Three-Dimensional Image Reconstruction in Radiology and Nuclear Medicine,* 2001, 70-73
- **L.A. FELDKAMP ; L.C. DAVIS ; J.W. KRESS.** Practical Cone-Beam ''Algorithm. *Journal Optical Society of America,* 1984, vol. 1, 612-619
- **DENNIS L. PARKER.** Optimal Short Scan Convolution Reconstruction for Fanbeam CT. *Med. Phys.,* 1982, vol. 9 (2
- Theory of Image Formation, Detection, and Processing. **H.H. BARRETT ; W. SWINDELL.** Radiological Imaging. Academic Press, 1981, vol. 2, 391-392
- **M. D. SILVER ; K. TAGUCHI ; K. S. HAN.** Field-of-view dependent helical pitch in multi-slice CT. *Proc. of SPIE Med. Imag. Conf.,* 2001, vol. 4320, 839-850
- **C. R. CRAWFORD ; K. F. KING.** Computed tomography scanning with simultaneous patient translation. *Med. Phys.,* 1990, vol. 17, 967-982
- **M. D. SILVER.** A method for including redundant data in computed tomography. *Med. Phys.,* 2000, vol. 27, 773-774
- **D. L. PARKER.** Optimal short scan convolution reconstruction for fanbeam CT. *Med. Phys.,* 1982, vol. 9, 254-257
- **S. K. PATCH ; A. NISHIDE ; A. HAGIWARA.** Volumetric computed tomography data weights - Resolution vs artifact. *Radiology,* 2002, vol. 225, 496